# EUROPEAN PATENT SPECIFICATION

(11) **EP 2 534 261 B1**
(45) Date of publication and mention of the grant of the patent: **30.11.2016**
(21) Application number: 11715264.5
(22) Date of filing: 11.02.2011
(51) Int. Cl.: C12Q 1/68

(54) **ASXL1 AS A NEW DIAGNOSTIC MARKER OF MYELOID NEOPLASMS**
ASXL1 ALS NEUER DIAGNOSEMARKER VON MYELOIDNEOPLASMEN
ASXL1 EN TANT QUE NOUVEAU MARQUEUR DIAGNOSTIQUE DE NÉOPLASIES MYÉLOÏDES

(30) Priority: 12.02.2010 US 303971 P
(43) Date of publication of application: 19.12.2012
(73) Proprietor: QIAGEN Marseille, 91940 Les Ulis (FR); Institut Paoli-Calmettes, 13009 Marseille (FR); INSERM - Institut National de la Santé et de la Recherche Médicale, 75654 Paris Cedex 13 (FR)
(72) Inventor: GELSI-BOYER, Véronique, F-13009 Marseille (FR); BIRNBAUM, Daniel, F-13720 La Bouilladisse (FR)
(74) Representative: Becker, Philippe
(86) International application number: PCT/IB2011/000255
(87) International publication number: WO 2011/098901

(56) References cited:
- WO-A1-2009/150229
- GELSI-BOYER VERONIQUE ET AL: "Mutations of polycomb-associated gene ASXL1 in myelodysplastic syndromes and chronic myelomonocytic leukaemia", BRITISH JOURNAL OF HAEMATOLOGY, vol. 145, no. 6, June 2009 (2009-06), pages 788-800, XP002637531, ISSN: 0007-1048
- CARBUCCIA N ET AL: "Mutations of ASXL1 gene in myeloproliferative neoplasms.", LEUKEMIA : OFFICIAL JOURNAL OF THE LEUKEMIA SOCIETY OF AMERICA, LEUKEMIA RESEARCH FUND, U.K NOV 2009 LNKD- PUBMED:19609284, vol. 23, no. 11, November 2009 (2009-11), pages 2183-2186, XP002637532, ISSN: 1476-5551
- CARBUCCIA N ET AL: "Mutual exclusion of ASXL1 and NPM1 mutations in a series of acute myeloid leukemias.", LEUKEMIA : OFFICIAL JOURNAL OF THE LEUKEMIA SOCIETY OF AMERICA, LEUKEMIA RESEARCH FUND, U.K FEB 2010 LNKD- PUBMED:19865112, vol. 24, no. 2, 29 October 2009 (2009-10-29), pages 469-473, XP002637533, ISSN: 1476-5551
- ABDEL-WAHAB OMAR ET AL: "Genetic Analysis of Transforming Events That Convert Chronic Myeloproliferative Neoplasms to Leukemias", CANCER RESEARCH, vol. 70, no. 2, January 2010 (2010-01), pages 447-452, XP002637534, ISSN: 0008-5472
- GELSI-BOYER VERONIQUE ET AL: "ASXL1 mutation is associated with poor prognosis and acute transformation in chronic myelomonocytic leukaemia", BRITISH JOURNAL OF HAEMATOLOGY, vol. 151, no. 4, November 2010 (2010-11), pages 365-375, XP002637535,
- KIYOI HITOSHI ET AL: "Prognostic implication of FLT3 and N-RAS gene mutations in acute myeloid leukemia", BLOOD, vol. 93, no. 9, 1 May 1999 (1999-05-01), pages 3074-3080, XP002637536, ISSN: 0006-4971
- B. L. STEIN ET AL: "Disruption of the ASXL1 gene is frequent in primary, post-essential thrombocytosis and post-polycythemia vera myelofibrosis, but not essential thrombocytosis or polycythemia vera: analysis of molecular genetics and clinical phenotypes", HAEMATOLOGICA, vol. 96, no. 10, 28 June 2011 (2011-06-28) , pages 1462-1469, XP055155571, ISSN: 0390-6078, DOI: 10.3324/haematol.2011.045591

## Description

### Field of the Invention

The present invention relates to genetic markers to diagnose myeloid neoplasms, more particularly to a new identified tumor suppressor gene. More particularly, ASXL1 is described as a marker, which is useful for diagnosing MDS, CMML, MPN and AML.

### Background

Hematopoiesis is maintained by a hierarchical system where hematopoietic stem cells (HSCs) give rise to multipotent progenitors, which in turn differentiate into all types of mature blood cells. The molecular mechanisms controling multipotentiality, self-renewal, quiescence and HSC commitment have been extensively studied. However, numerous issues remain to be addressed and important genes regulating these processes remain to be identified.

Acute Myeloid Leukemia (AML), Myeloproliferative neoplasms (MPNs), myelodysplastic syndromes (MDS) and myelodysplastic/myeloproliferative disorders are clonal stem-cell malignant disorders.

Several genetic mutations have been correlated to AML, and four groups are recognized: (i) AMLs with recurrent genetic abnormalities AML t(8;21)(q22;q22) with RUNX1-ETO fusion gene; AML with abnormal bone marrow eosinophils and inv(16)(p13;q22) or t(16;16)(p13;q22) with CBFB/MYH1 1 rearrangement; acute promyelocyte leukaemia APL with t(15;17)(q22;q12) PML/RARA; AML with 11q23 (MLL) abnormalities); (ii) AML with multilineage dysplasia following MDS or MDS/MPNor without antecedent of MDS or MPN; (iii) AML or MDS therapy related and (iv) other unclassified AMLs, which comprise the group of AMLs with normal karyotype whose prognosis is based on molecular analysis of oncogenes such as mutations of FLT3-ITD or NPM1.

Myelodysplastic/myeloproliferative neoplasms include four myeloid diseases grouped in 1999 by the WHO: chronic myelomonocytic leukemia (CMML), juvenile myelomonocytic leukemia (JMML), atypical chronic myeloid leukemia (aCML) and unclassified myelodysplastic/myeloproliferative syndromes (U-MDS MPS).

MPNs include chronic myelogenous leukemia (CML), polycythemia vera (PV), essential thrombocytopenia (ET) and idiopathic myelofibrosis (IMF). MPNs are characterized by an increased proliferation of one or several myeloid lineages and are commonly associated with an acquired constitutive kinase activity, as exemplified by the JA 2V617F mutation in Polycythemia Vera.

MDS are classified into several classes including refractory anemia (RA), and refractory cytopenia with multilineage dysplasia (RCMD), and RA with excess of blasts (RAEB). MDS are characterized by ineffective hematopoiesis in one or more of the lineage of the bone marrow, but the underlying molecular defects are still poorly understood. No biological markers, except morphological features, are currently available for early diagnosis and prognosis.

The ASXL (additional sex combs) family of genes has three members in humans encoding poorly characterized proteins containing a C-terminal PHD finger (plant homeodomain). It is now expected that these ASXL proteins regulate chromatin remodeling and are potentially linked to regulation of transcription.

More specifically, the ASXL1 (additional sex combs like 1) gene (also known as IAA0978; MGC711 1 1; MGC117280) is located on the chromosomal region 20q11, comprises 12 exons over about 1OOkb. This gene is referenced under the accession number ID 171023 and its cDNA (Accession number NM 015338, SEQ ID N°1) encodes a protein of 1541 amino acids (Accession number NP 056153, SEQ ID N°2).

The ASXL1 protein helps recruiting polycomb and thrithorax complexes to specific domains and shares four conserved domains consisting in i) the ASXN domain (amino acids 1 to 86), ii) the ASXM (amino acids 250 to 361), iii) the NR box (the amino acids 1 107 to 1 1 12) and i) the PHD domain (amino acids 1506 to 1541, SEQ ID N°3). The ASXL1 protein plays a role in transcription regulation of differentiation (e.g. retinoic acid pathway) and self-renewal programs.

Gelsi-Boyer et al, 2009, British Journal of Haematology, 145(6):788-800, reports mutations of ASXL1 gene in myelodysplastic syndromes and chronic myelomonocytic leukaemia. Carbuccia et al, 2009, Leukemia, 23(11):2183-2186, and Carbuccia et al, 2009, Leukemia, 24(2):469-473, disclose ASXL1 mutations in myeloproliferative neoplasms. Abdel-Wahab et al, 2010, Cancer Research, 70(2):447-452, is concerned with the transformation of chronic myeloproliferative neoplasm (MPN) to acute myeloid leukemias (AML).

### Summary of the Invention

It is described mutations of ASXL1 gene in myelodysplasia syndromes (MDS), in myelodysplastic/myeloproliferative neoplasms (i.e. chronic myelomonocytic leukemia (CMML)), in MPNs, and in Acute Myeloid Leukemia (AML).

The invention is as defined in the claims.

It is further described a method for diagnosing a myeloid cancer in a subject, which comprises the step of analyzing a biological sample from said subject by determining the presence or the absence of a mutation in the ASXL1 (additional sex combs like 1) gene coding for the polypeptide having the sequence SEQ ID N°2, wherein the presence of such a mutation is correlated with a myeloid cancer. Identification of the presence or the absence of the mutation may be performed by comparison to a normal control, e.g. a cell line, which does not comprise said mutation. The method may further comprise the step of recording the presence or absence of said mutation at a particular position.

In a particular aspect, said method is for diagnosing a myeloproliferative neoplasm (MPN) in a subject, preferably said MPN is a primary myelofibrosis (PMF), post-polycythemia vera myelofibrosis (post-PV MF) or post essential thrombocythemia myelofibrosis (post-ET MF).

Advantageously, said mutation is selected in the group consisting of insertions, deletions, and point mutations corresponding to missense mutation and nonsense mutations, preferably in the group consisting of insertions, deletions, and nonsense mutations.

Preferably, said mutation results in the expression of a mutated ASXL1 protein, said mutated ASXL1 protein does not comprises any longer its Plant HomeoDomain (PHD domain, SEQ ID N°3) or a fragment thereof.

It is also described a kit for diagnosing myeloid cancer in a subject comprising at least one nucleic acid probe or oligonucleotide or at least one antibody, which can be used in a method as defined previously for determining the presence or the absence of a mutation in the ASXL1 (additional sex combs like 1) gene coding for the polypeptide having the sequence SEQ ID N°2, wherein the presence of such a mutation is correlated with a myeloid cancer.

It is further described a method for the prognosis of the outcome of a myeloid cancer in a subject, which comprises the step of analyzing a biological sample from said subject by determining the presence or the absence of a mutation in the ASXL1 (additional sex combs like 1) gene coding for the polypeptide having the sequence SEQ ID N°2, wherein the presence of the mutation is indicative of a poor prognosis of said patient, and the absence of the mutation is suggestive of a good prognosis of said patient. Mutations that are suitable for said prognosis include, but are not limited to, disclosed mutations in the ASXL1 gene. The presence or the absence of the mutation may be performed by comparison to a normal control, e.g. a cell line, which does not comprise said mutation. The method may further comprise the step of recording the presence or absence of said mutation at a particular position.

In a first aspect, said myeloid cancer is a polycythemia vera (PV) and the method of the invention is for the prognosis of the progression of said PV to post-polycythemia vera myelofibrosis (post-PV MF). The presence of an ASXL1 mutation is indicative of a risk of a progression of said PV to Post-PV MF.

In another aspect, said myeloid cancer is an essential thrombocythemia (ET) and the method of the invention is for the prognosis of the progression of said PV to post-essential thrombocythemia myelofibrosis (post-ET MF). The presence of an ASXL1 mutation is indicative of a risk of a progression of said ET to Post-PV ET.

### Brief Description of the Drawings

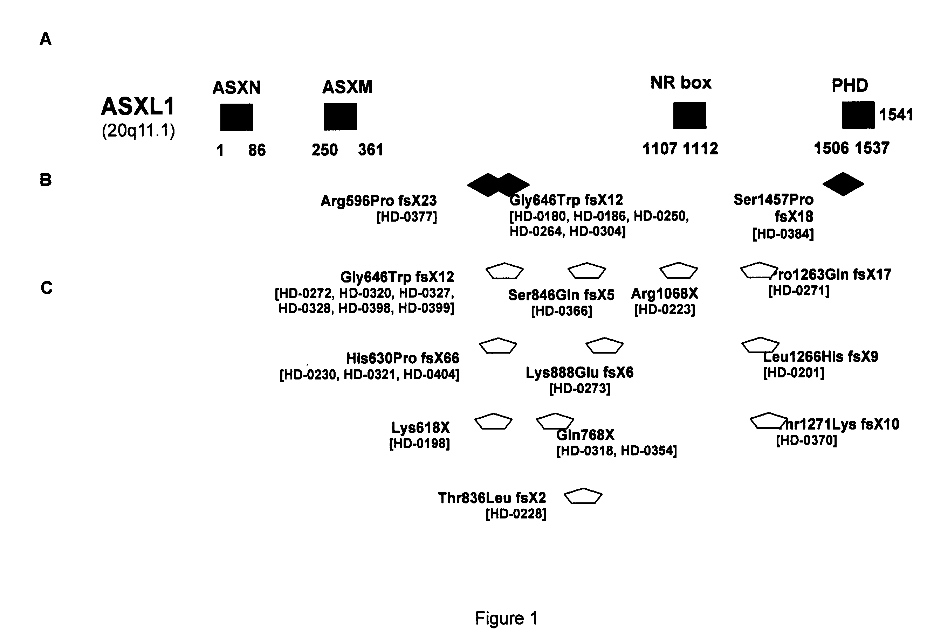

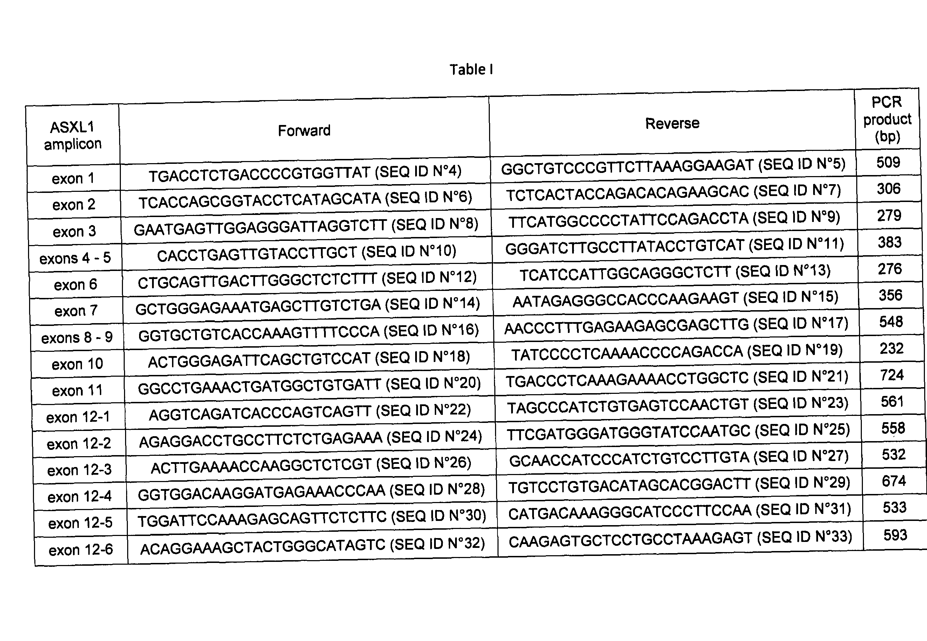

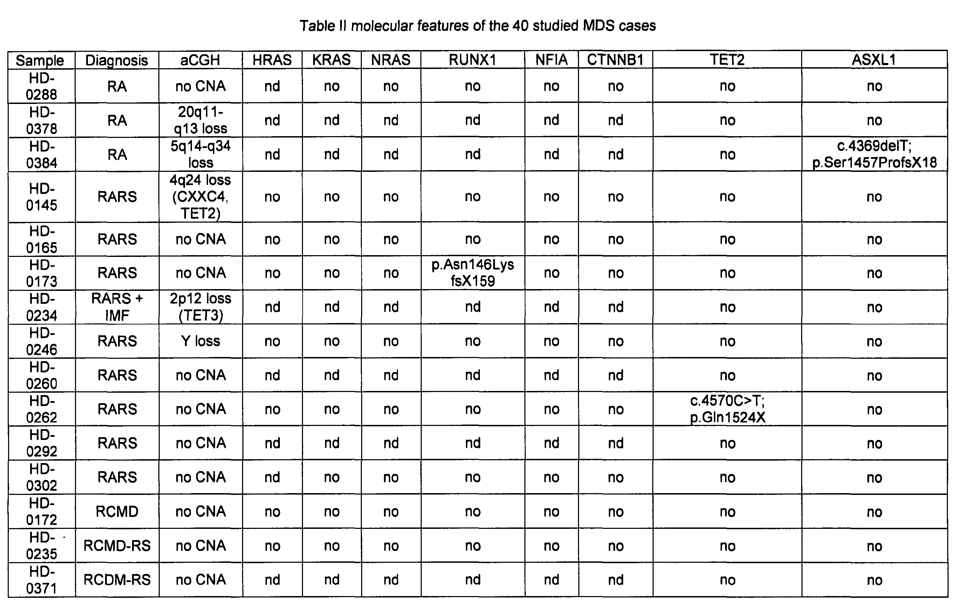

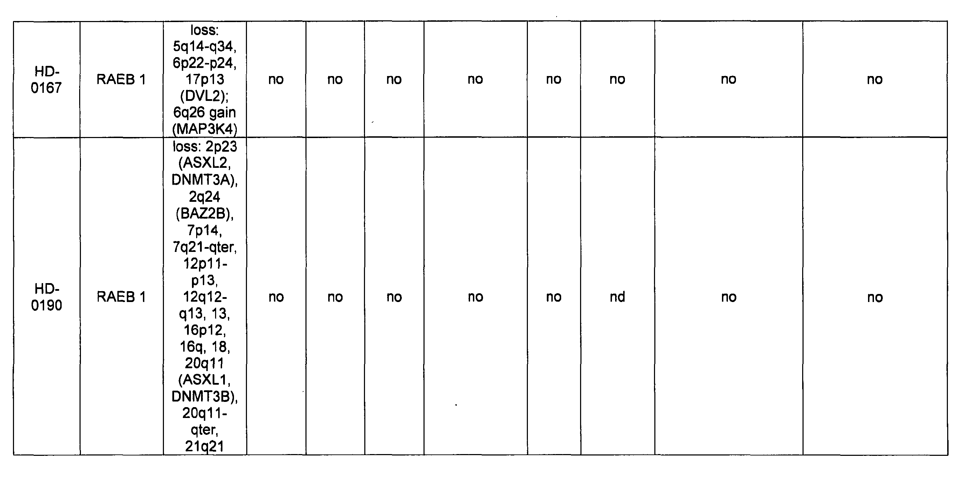

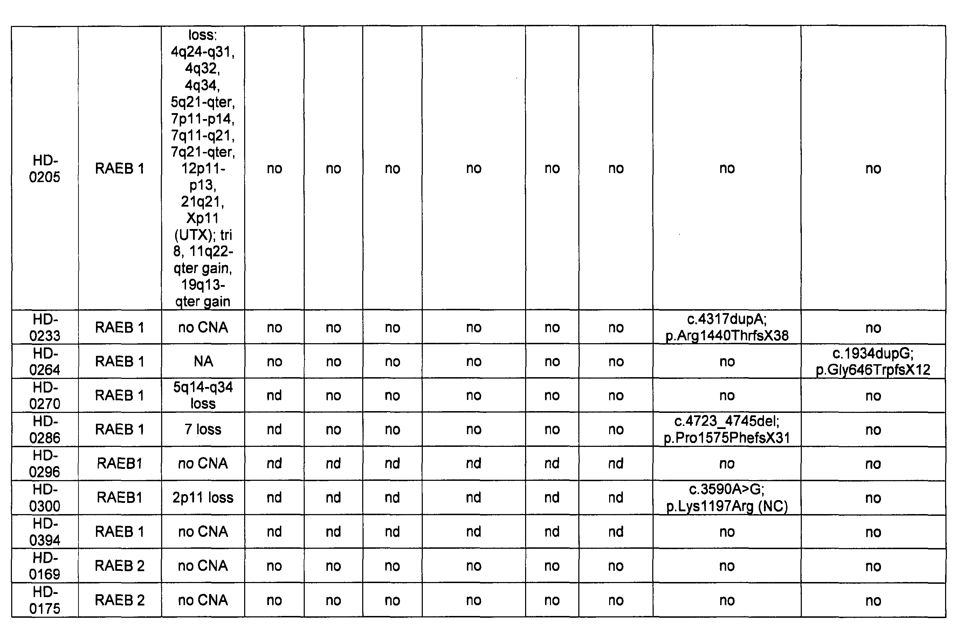

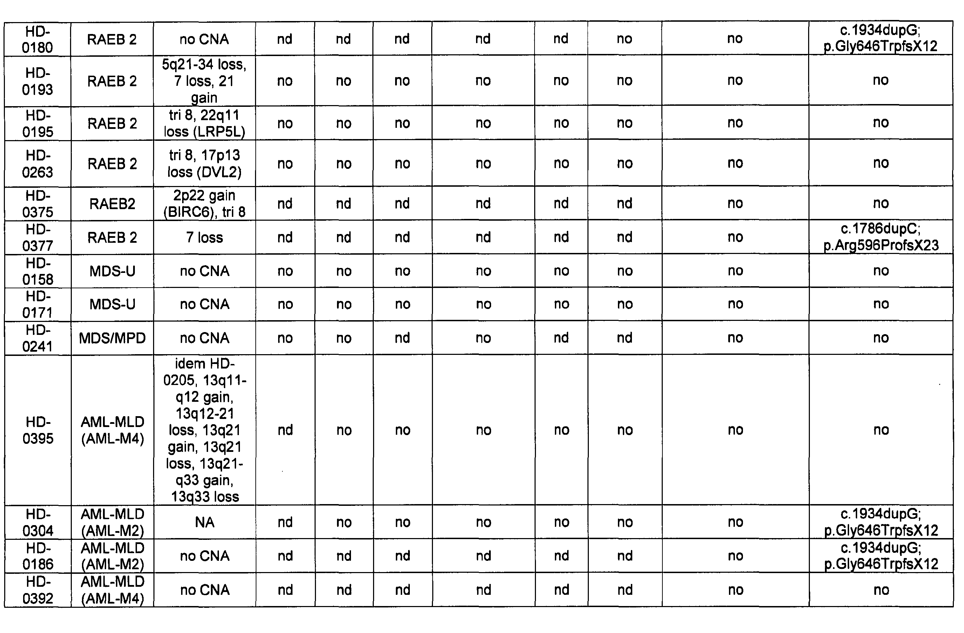

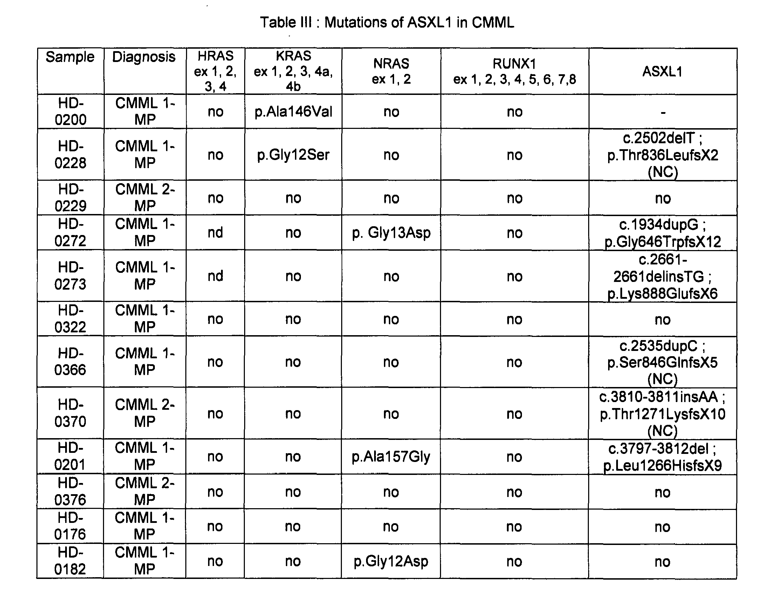

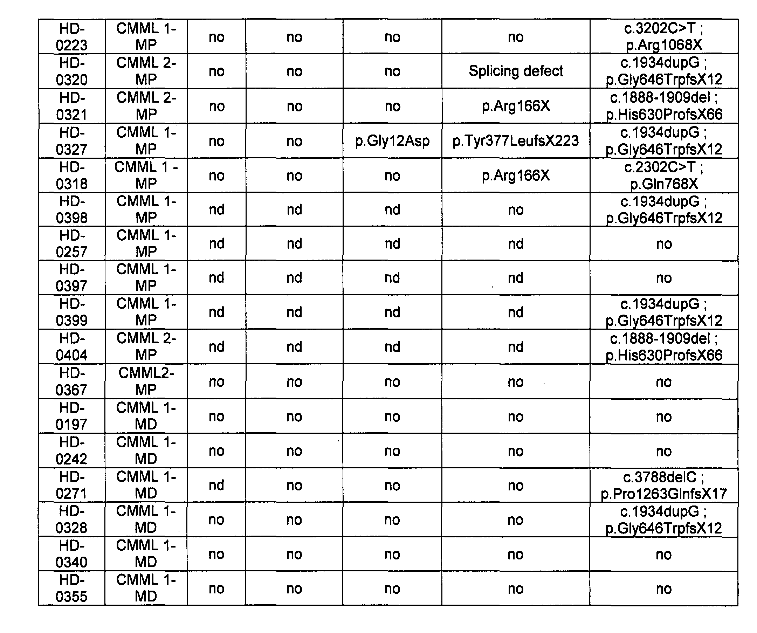

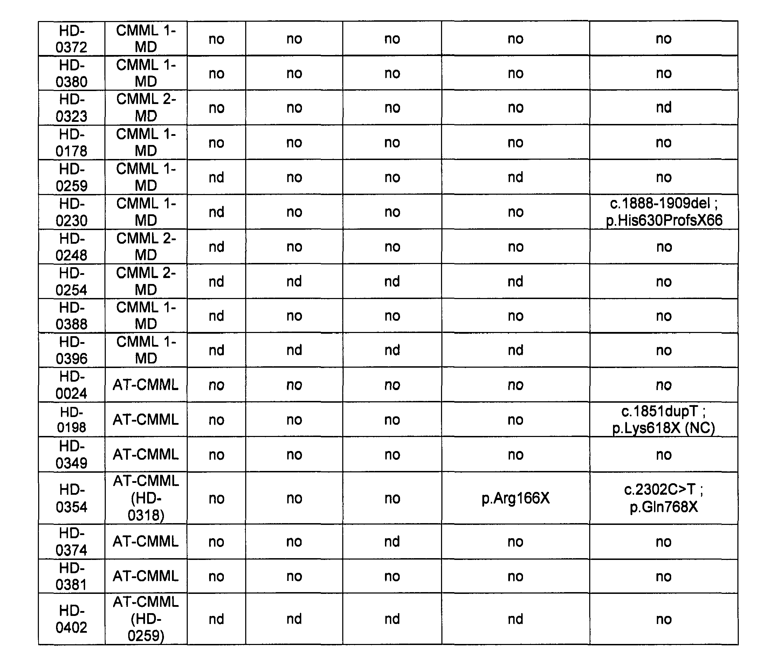

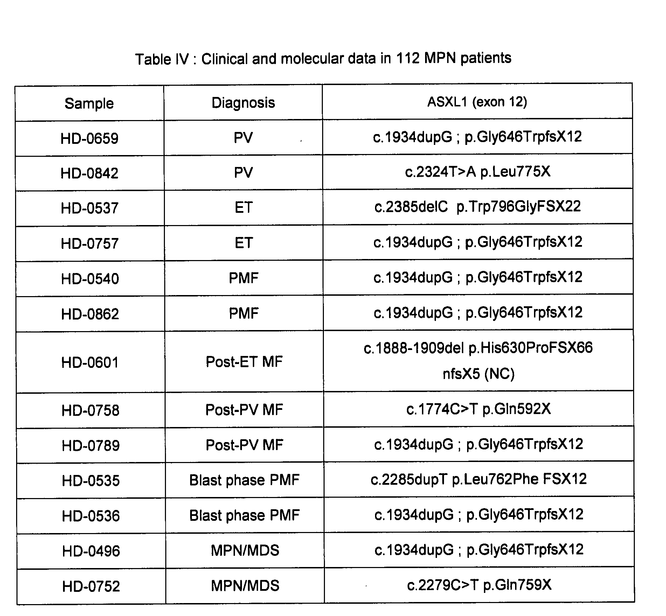

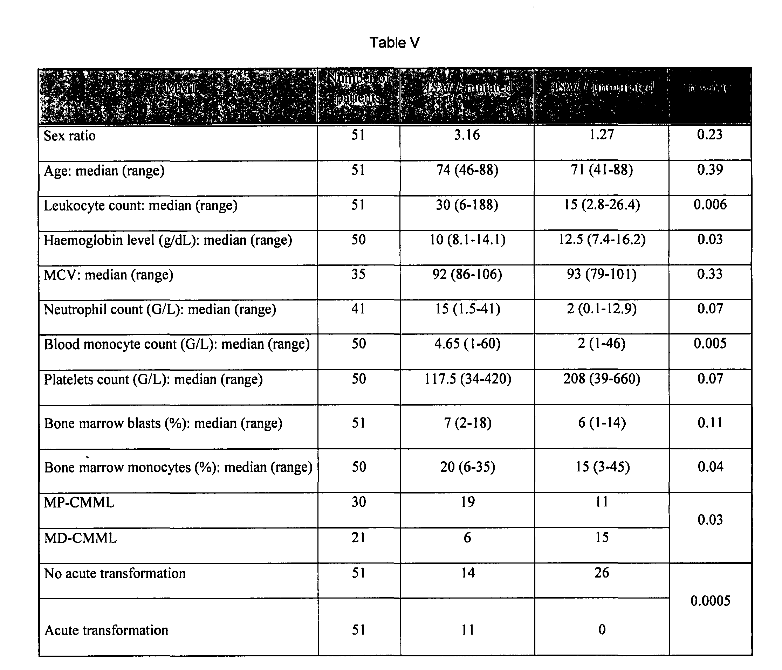

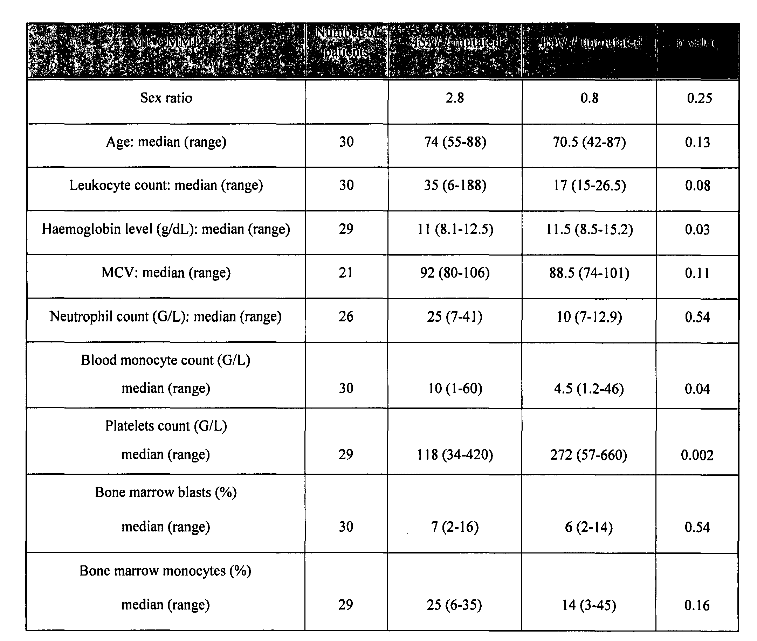

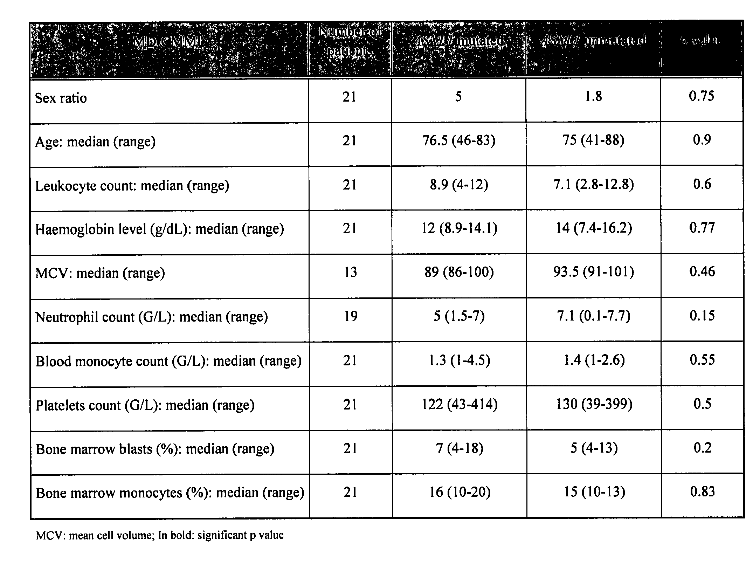

Figure 1 shows the representation of the ASXL1 protein with known motifs and domains.
Table I shows the pairs of primers sequences used to amplify and sequence the ASXL1 gene.
Table II shows the molecular features of 40 studied MDS cases.
Table III shows the mutations of ASXL1 in CMML.
Table IV shows the clinical and molecular data of 1 12 MPNs patients with ASXL1 mutations.
Table V shows the clinical and biological features of ASXL1 mutated and unmutated CMML patients.
Figure 2 shows the Kaplan-Meier overall survival curves of CMML patients according to ASXL1 mutational status.
Figure 3 shows the Kaplan-Meier overall survival curves of CMML patients according to ASXL1 mutational status in patients whose disease had not evolved in acute leukaemia.

### Detailed Description

The present invention is based on the discovery by the present inventors that the ASXL1 gene is often targeted by mutations and/or deletions in tumoral cells in patients sufferings from MDS, myelodysplatic/myeloproliferative neoplasms, MPN or AML.

It is described a method for diagnosing a myeloid cancer in a subject, which comprises the step of analyzing a biological sample from said subject by determining the presence or the absence of a mutation in the ASXL1 (additional sex combs like 1) gene coding for the polypeptide having the sequence SEQ ID N°2, wherein the presence of such a mutation is correlated with a myeloid cancer.

As used herein, the term "subject" refers to a mammal, preferably a human.

Said subject may be healthy, but the method herein described is particularly useful for testing a subject thought to develop or to be predisposed to developing a myeloid cancer. In that case, the method enables to confirm that said subject develops or is predisposed for developing a myeloid cancer.

Said method is for diagnosing a myeloproliferative neoplasm (MPN) in a subject.

In fact, the inventors have established on a MPN patients panel that at least 8% of them comprise an ASXL1 mutation.

Preferably, said MPN is a primary myelofibrosis (PMF) since the inventors have established on a PMF patients panel that at least 33% of them comprise an ASXL1 mutation.

Still preferably, said MPN is post-polycythemia vera myelofibrosis (post-PV MF) since the inventors have established that at least 66% of them comprised an ASXL1 mutation.

Still preferably, said MPN is post-essential thrombocythemia myelofibrosis (post-ET MF) since the inventors have established that at least 25% of them comprised an ASXL1 mutation.

Still preferably, said MPN is essential thrombocythemia (ET) and the presence of a mutation in the ASXL1 gene exclude a reactive thrombocytosis (RT) since the inventors have established that reactive thrombocytosis patients do not share any ASXL1 mutation.

As used herein, the expression "biological sample" refers to solid tissues such as, for example, a lung biopsy; buccal swab, fluids and excretions such as for example, sputum, induced sputum, blood, serum, plasma, urine. Preferably, said biological sample is a blood or bone marrow sample, preferably a bone marrow sample. Preferably, only a biological sample containing cells including genomic DNA (or optionally RNA) from the subject to be tested is required.

As used herein, the term "mutation" corresponds to any modification in the sequence of the original nucleic acid sequence. These mutations comprise small-scale mutations, or large scale mutations. Small scale mutations are those affecting a gene in one or a few nucleotides, including point mutations, insertions or deletions of one or more extra nucleotides in the DNA. Point mutations can be silent, missense and nonsense mutation. Large scale mutation in the genomic structure, such as gene duplications, deletions, or mutations whose effect is to juxtapose previously separate pieces of DNA, potentially bunging together separate genes to form functionally distinct fusion genes.

Preferably, said mutation is selected in the group consisting of insertions, deletions, and point mutations corresponding to missense mutation and nonsense mutations.

More preferably, said mutation is selected in the group consisting of insertions, deletions, and nonsense mutations.

Moreover, the inventors have established that the exon 12 of the gene encoding the PHD domain of the ASXL1 protein is preferentially targeted by the deleterious mutations in the studied patients {See examples).

Thus, and still preferably, said mutation results in the expression of a mutated ASXL1 protein that does not comprise any PHD domain (SEQ ID N°3) or a fragment thereof.

Said mutated protein can result from the introduction of a non sense mutation leading to the introduction of a stop codon (X) in the open reading frame of the ASXL1 protein. As an example, said non-sense mutation is selected in the group comprising Tyr591X, Gln592X, Lys618X, Arg693X, Gln759X, Gln768X, Leu775X and Arg1068X.

Said mutated protein can also result from a frame-shift (FS) because of an insertion of a deletion in the ASXL1 gene. As an example, said insertion or deletion is selected in the group of the ones inducing the expression of the mutated ASXL1 protein with the following mutations Gly64Trp FS, Arg596Pro FS, Ala61 1Arg FS, His630Pro FS, Gly646Trp FS, Leu762Phe FS, Trp796Gly FS, Thr822Asn FS, Thr836Leu FS, Ser846Gln FS, Asp879Glu FS, Lys888Glu FS, Leul213Ile FS, Prol263Gln FS, Leul266His FS, Trpl271Lys FS, or Serl457Pro FS.

In another preferred embodiment of the invention, the determining step is done on genomic DNA.

Typical techniques for detecting the presence of a mutation in DNA may include restriction fragment length polymorphism, hybridization techniques, DNA sequencing, exonuclease resistance, microsequencing, solid phase extension using ddNTPs, extension in solution using ddNTPs, oligonucleotide ligation assays, methods for detecting single nucleotide polymorphisms such as dynamic allele-specific hybridization, ligation chain reaction, mini-sequencing, DNA "chips", high resolution melting (HRM) method, amplification-refractory mutation system (ARMS) method, allele-specific oligonucleotide hybridization with single or dual-labelled probes merged with PCR or with molecular beacons, Scorpions® probes (DxS Genotyping), MGB® (Minor Groove Binding) probes (NANOGEN) and others.

Advantageously, the mutation is detected on the cDNA of the ASXL1 gene by either PCR and sequencing, SNP-array or CGH, all of them being well known for the skilled person.

Comparative genomic hybridization (CGH) is a molecular cytogenetic method of screening a tumor for genetic changes. The alterations are classified as DNA gains and losses and reveal a characteristic pattern that includes mutations at chromosomal and subchromosomal levels. The method is based on the hybridization of fluorescently labeled tumor DNA (frequently fluorescein (FITC)) and normal DNA (frequently rhodamine or Texas Red) to normal human metaphase preparations. Using epifluorescence microscopy and quantitative image analysis, regional differences in the fluorescence ratio of gains/losses vs. control DNA can be detected and used for identifying abnormal regions in the genome. CGH will detect only unbalanced chromosomes changes. Structural chromosome aberrations such as balanced reciprocal translocations or inversions can usually not be detected, as they do not systematically change the copy number.

In another preferred embodiment of the invention, the determining step is realized on ASXL1 mRNA/cDNA.

Such analysis can be assessed by preparing mRNA/cDNA from cells in a biological sample from a subject, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TAQMAN), and probes arrays such as GENECHIP DNA Arrays (AFFYMETRIX).

In still another preferred embodiment of the invention, the determining step is realized on the ASXL1 protein.

Such analysis can be assessed using an antibody {e.g., a radio-labeled, chromophore-labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, etc.) which binds specifically to the protein translated from the ASXL1 gene (SEQ ID N°2), and preferably to the PHD domain (SEQ ID N°3) of the ASXL1 protein.

Said analysis can be assessed by a variety of techniques well known by one of skill in the art including, but not limited to, enzyme immunoassay (EIA), radioimmunoassay (RIA), Western blot analysis and enzyme linked immunoabsorbant assay (ELISA).

Polyclonal antibodies can be prepared by immunizing a suitable animal, such as mouse, rabbit or goat, with the ASXL1 protein (SEQ ID N°2) or the PHD domain thereof (SEQ ID N°3).The antibody titer in the immunized animal can be monitored over time by standard techniques, such as with an ELISA using immobilized polypeptide. At an appropriate time after immunization, e.g., when the specific antibody titers are highest, antibody producing cells can be obtained from the animal and used to prepare monoclonal antibodies (mAb) by standard techniques.

The skilled person can also use commercially available ASXL1 monoclonal antibodies, such as the monoclonal antibodies commercialized by ABCAM or by SANTA CRUZ BIOTECHNOLOGY Inc.

It is further described a kit for diagnosing myeloid cancer in a subject comprising at least one nucleic acid probe or oligonucleotide or at least one antibody, which can be used in a method as defined previously, for determining the presence or the absence of a mutation in the ASXL1 (additional sex combs like 1) gene coding for the polypeptide having the sequence SEQ ID N°2, wherein the presence of such a mutation is correlated with a myeloid cancer.

Preferably, the oligonucleotide is at least one PCR primer, preferably a set of PCR primers is provided, which allows to amplify the ASXL1 gene or a fragment thereof. The skilled person readily provides such an oligonucleotide or set of PCR primers which allows to amplify a region of the ASXL1 gene, provided that the nucleic acid sequence of the ASXL1 gene is well known (Accession number NC 000020.10, nucleotides 30,946,153 to 31,027,122; and accession number NM_015338 for the corresponding cDNA, SEQ ID N°1).

As an example, said pairs of PCR primers are selected in the group comprising SEQ ID N°4 and SEQ ID N°5, SEQ ID N°6 and SEQ ID N°7, SEQ ID N°8 and SEQ ID N°9, SEQ ID N°10 and SEQ ID N°11, SEQ ID N°12 and SEQ ID N°13, SEQ ID N°14 and SEQ ID N°15, SEQ ID N°16 and SEQ ID N°17, SEQ ID N°18 and SEQ ID N°19, SEQ ID N°20 and SEQ ID N°21, SEQ ID N°22 and SEQ ID N°23, SEQ ID N°24 and SEQ ID N°25, SEQ ID N°26 and SEQ ID N°27, SEQ ID N°28 and SEQ ID N°29, SEQ ID N°30 and SEQ ID N°31, and SEQ ID N°32 and SEQ ID N°33. Such primers are disclosed in table I.

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte Specific Reagents (ASRs) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

The present kits can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugatable markers, for example biotin and streptavidin or the like.

In one embodiment, the kit is made up of instructions for carrying out the method described herein for diagnosing a myeloid cancer in a subject. The instructions can be provided in any intelligible form through a tangible medium, such as printed on paper, computer readable media, or the like.

It is described a method for the prognosis of the outcome of a myeloid cancer in a subject, which comprises the step of analyzing a biological sample from said subject by determining the presence or the absence of a mutation in the ASXL1 (additional sex combs like 1) gene coding for the polypeptide having the sequence SEQ ID N°2, wherein the presence of the mutation is indicative of a poor prognosis of said patient, and the absence of the mutation is suggestive of a good prognosis of said patient. Mutations that are suitable for said prognosis include, but are not limited to, disclosed mutations in the ASXL1 gene. The presence or the absence of the mutation may be performed by comparison to a normal control, e.g. a cell line, which does not comprise said mutation. The method may further comprise the step of recording the presence or absence of said mutation at a particular position.

In an aspect, said myeloid cancer is a polycythemia vera (PV) and the method of the invention is for the prognosis of the progression of said PV to post-polycythemia vera myelofibrosis (post-PV MF). The presence of an ASXL1 mutation is indicative of a risk of a progression of said PV to Post-PV MF.

In a further aspect, said myeloid cancer is an essential thrombocythemia (ET) and the method of the invention is for the prognosis of the progression of said PV to post-essential thrombocythemia myelofibrosis (post-ET MF). The presence of an ASXL1 mutation is indicative of a risk of a progression of said ET to Post-PV ET.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### EXAMPLES

### 1) Alteration of the ASXL1 gene in patients suffering from MDS

### Three types ofaCHG profiles in MDSs

A series of bone marrow (BM) samples were collected from patients with MDS, with AML with multilineage dysplasia (AML-MLD), and with AML secondary to CMML.

According to the French-American- British (FAB) and WHO criteria, the MDS panel comprised three RA, nine RARS (including one with idiopathic myelofibrosis), three RCMD (including two with ring sideroblasts), 10 RAEB1, eight RAEB2 and two MDS-U.

The majority of MDS samples were collected at the time of diagnosis; some were in therapeutic abstention of a known MDS and some were under symptomatic treatment. All were *de novo except* two cases secondary to treatment for solid tumours.

Genome-wide high-density arrays were used to study the aCGH profiles of 40 MDS/AML samples from 38 patients.

The DNA was extracted by the ALLPREP DNA/RNA isolation kit MACHEREY NAGEL from total bone marrow cells, as recommended by the supplier.

DNA imbalances were analysed by Array comparative genomic hybridization (aCGH) using 244K CGH MICROARRAYS (Hu-244A; AGILENT TECHNOLOGIES) an the résolution was up to 6kb. Scanning was done with Agilent Autofocus Dynamic Scanner (G2565BA; AGILENT TECHNOLOGIES). Data analysis was made as previously described in GELSI-BOYER et al. (BMC Cancer, vol.8, p :299-314, 2008) and visualized with CGH ANALYTICS 3.4 software (AGILENT TECHNOLOGIES). Extraction data (log₂ ratio) was done with CGH analytics while the normalized and filtered log₂ ratios were obtained from 'FEATURE EXTRACTION' software (AGILENT TECHNOLOGIES). Copy number changes were characterized as reported in GELSI-BOYER *et al* (abovementioned, 2008).

The results are summarized in Table II.

In these results, tree main types of profiles were observed.

Type 1 profiles showed gains or losses that were already visible on the karyotype and affected large regions of the genome, such as trisomy 8, deletions of part of the 5q and 20q arms, or deletion or complex rearrangements of chromosome 7. Deletions on 5q arm were quite large and always comprised *RPS14, HSPA9B* and many other genes, including *CXXC5* (CXXC finger 5).

Type 2 profiles showed rare and limited gains or losses that affected few genes. On case (i.e. case 190) showed several regions with small deletions. One of these at 20q11 contained the *ASXL1* (additional sex combs 1) and *DNMT3B* (DNA cytosine-5-methyltransferase 3 beta) genes and another one at 2p23 the *ASXL2* and *DNMT3A* paralogous genes. The same case also showed a deletion of *BAZ2B* on chromosome arm 2q. Gains were also observed (i.e. MAP3K4 in case 167) but less frequently than deletions.

In cases with a type 3 profile no genomic copy number aberration (CNA) could be detected. This profile was found in 22/40 cases (55%). We have indicated this by 'no CNA' in Table I.

### Mutations of candidate genes in MDSs

We analysed the sequences of several candidate genes in our MDS samples.

Somatic mutations of HRAS, KRAS, NRAS, RUNX1, NFIA, CTNNB1, TET2 and ASXL1 genes were searched by sequencing exons and consensus splicing sites after polymerase chain reaction (PCR) amplification of genomic DNA (See Table I for ASXL1). PCR amplifications were done in a total volume of 25 µl PCR mix containing at least 5 ng template DNA, Taq buffer, 200 µmol of each deoxynucleotide triphosphate, 20 pmol of each primer and 1 unit of HOT STAR TAQ (QIAGEN).

PCR amplification conditions were as follows: 95°C 10 min; 95°C 30 s, 55°C 30 s, 72°C 30 s to 1 min depending on PCR product length for 35 cycles; 72°C 10 min.

PCR products were purified using MILLIPORE PLATE MSNU030 (MILLIPORE SAS). Aliquots (1 µl) of the purified PCR products were used for sequencing using the BIG DYE TERMINATOR V1.1 kit (APPLIED BIOSYSTEMS) including the forward or reverse primer.

After G50 purification, sequences were loaded on an ABI 3130XL AUTOMAT (APPLIED BIOSYSTEMS). The sequence data files were analysed using the SEQSCAPE software and all mutations were confirmed on an independent PCR product.

The results are presented in Table I and show that no mutation of the three RAS genes, of the NFIA gene or of the CTNNB1 gene was found.

RUNX1 mutation was found in one case out of 24 tested.

We found several cases mutated for TET2 (to be reported in detail elsewhere). Mutations and deletions of this gene have been discovered recently in about 15-20% of various myeloid diseases including MDSs.

We searched for mutations in the ASXL1 gene, one allele of which was deleted in case 190. We found six mutations in five patients (one of these mutations was found in both the MDS and transformed states) (Table II). The mutations were caused by deletion or duplication of a nucleotide. Figure 1A and B shows a schematic representation of the ASXL1 protein with the deduced localization of the mutations. The mutations were all found in exon 12 of the gene and should lead to the truncation of the C-terminus of the protein, which contains a PHD finger.

Finally, e thus found mutations in the *ASXL1* gene in 11% of MDS patients.

### 2) Alteration of the ASXL1 gene in patients suffering from Myelodysplastic/myeloproliferative disorders

### Mutation of ASXL1 in CMML

To determine whether ASXL1 mutations can be found outside MDSs we analysed as described previously the ASXL1 sequence in the bone marrow samples of patients suffering from CMML, a related disease.

According to the FAB and WHO criteria, the series of CMML comprised 21 myeloproliferative (MP-CMML), 18 myelodysplastic (MD-CMML) forms and seven acutely- transformed CMMLs (AT-CMML). All the patients signed an informed consent. The project and collection of samples were reviewed by the independent scientific review board of the Paoli-Calmettes Institute, in accordance with current regulations and ethical concerns.

A total of 19 mutations were found in 44 patients (46 cases) (43%) (Table III). The localization and nature of these mutations are shown in Figure 1C.

Like in MDS, all the mutations were found in exon 12 and were deletions, duplications, insertions or substitutions of nucleotides. We found 13 mutations in 21 MP-CMML (62%), four in 18 MD-CMML (22%) and two in seven AT-CMML (28%) cases, the difference between MP and MD cases being significant.

### Correlations between ASXL1 mutation and clinical and biological features in CMML

A series of consecutive bone marrow samples obtained from 53 patients, who all signed an informed consent, were collected. Among these 31 were MP-CMML and 22 were MD-CMML as initially defined by the FAB group with a leukocyte count superior or inferior to 13G/L, respectively. A normal karyotype was observed in 40 patients (20 MP-CMML and 20 MD-CMML); a del(20q)(q11;q13) was found in 3 patients (2 MP-CMML and 1 MD-CMML); a trisomy of a commonly affected chromosomes (8, 19, 21) was encountered in 4 MP-CMML. One MD-CMML had an 11q inversion, one MP-CMML had a t(10;11)(p12;p15) and one MP-CMML had a t(1;3)(p36;q21).

The aCGH profiles of 51 of the 53 CMML cases were established as described previously and revealed alterations that were observed by conventional cytogenetics (9/51) except for 3 patients with balanced translocations (HD-0201, HD-0316, HD-0178), and for case HD-0367 with a del(20)(q11q13) for which aCGH did not show a frank deletion at 20q probably due to the low number of affected cells.

For nine cases (17%) aCGH detected rare and limited losses or gains, not visible on the karyotype. They affected very few genes including some with known tumor suppressor function and leukaemogenic activity (*NF1, RB1* and *TET2).* Finally and in 70% of cases (36/51) no copy number aberrations were observed.

The results show also that the genomic alterations detected by conventional cytogenetics or aCGH were different in MP- and MD-CMMLs with 15 alterations out of 31 MP-CMMLs and 4 alterations out of 22 MD-CMMLs. Thus, MP-CMMLs had more genomic alterations than MD-CMMLs (p=0.049).

We studied coding sequences of 13 genes on the 53 cases. In 25 cases (49%) we found 20 frameshift (including 7 times the same p.Gly646Trpfsx12) and 5 nonsense mutations in *ASXL1* exon 12. *CBL* exon 8 mutations were found in 10% of cases (5/47). One case (HD-0223) had a homozygous deletion. One case (HD-0367) had an internal tandem duplication of *FLT3.* We found 5 *IDH* mutations in 48 cases (10%); all were in *IDH2* (4 times the same p.Arg140Gln). Seven patients out of the 53 cases had a K or *NRAS* mutation (13%). Twelve out of 53 patients (21%) were mutated for *RUNX1* and 36% of patients were mutated for *TET2.* No mutation was found in *NPM1, JAK2* and *WT1*.

We then studied the prevalence of the mutated genes in MP and MD-CMML. Nineteen of the 25 *ASXL1* mutations were found in 30 MP-CMML vs. 6 in 22 MD-CMML. Mutations in *ASXL1* but not in *RUNX1* or *TET2* were more frequent in MP than in MD-CMMLs (p=0.03). No difference was observed between the two forms for *CBL, FLT3, IDH*1l2, *PTPN11*, *RAS, RUNX1* or *TET2.* Overall the number of mutations (*ASXL1* and proliferation genes) was higher in MP (69/273 events) than in MD-CMMLs (26/172) (p=0.0018).

Since the classification of CMML has always been a matter of debate, thus the *ASXL1* mutation corresponds to a molecular basis to the separation of CMML in MP and MD forms initially defined by the FAB group.

In the present study *ASXL1* appeared as the most frequently mutated gene in CMML, as it is in MDSs.

The main clinical and biological features of 51 CMML cases were examined with respect to *ASXL1* mutations (Table V).

The results shown that the presence of an *ASXL1* mutation was associated with higher WBC (30 g/L vs. 15 g/L) (p=0.006), higher blood (p=0.005) and bone marrow monocytosis (p=0.04) and with lower level of blood haemoglobin (p=0.03). No difference was noted in mean cell volume, blood count of neutrophils and platelets, or bone marrow blasts. In MP-CMML *ASXL1* mutation correlated with a lower level of haemoglobin (p=0.03) and platelet count (p=0.002) and with a higher monocytosis (p=0.04). In MD-CMML, no correlations with *ASXL1* mutation were observed.

Among *ASXL1* mutated cases (25/51), eleven (9 MP and 2 MD) had evolved to acute transformation (Table V), whereas no acute transformation was observed in the unmutated cases. In other words, all transformed cases had an *ASXL1* mutation but not all *ASXL1* mutated cases had progressed to AML.

*ASXL1* mutation and acute transformation were thus correlated (p=0.0005). If some mutated cases had not progressed to acute phase, this may be because patients had died before experiencing acute leukaemia.

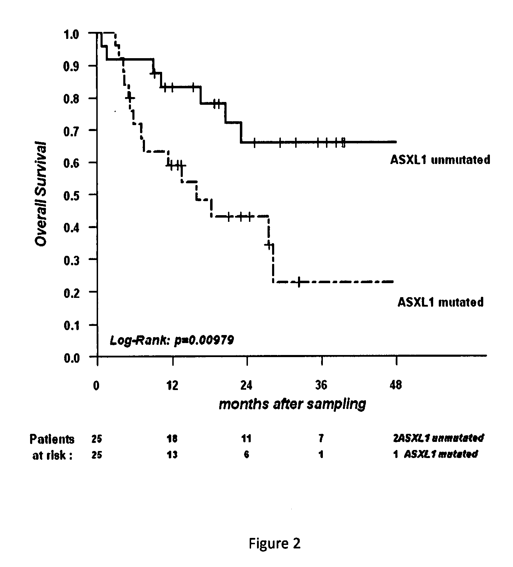

Analysis of overall survival (median follow-up of 29.5 months) was done for the 53 patients and the determined median overall survival was 27.6 months. *ASXL1* status could be determined at the time of sampling for 51 patients. Kaplan-Meier analysis showed a lower overall survival rate in the *ASXL1* mutated patients (Fig 2), with no significant impact of acute transformation on overall survival (data not shown). The results have also shown that with respect to MP/MD form, only a trend to a better survival of the MD patients was observed (data not shown).

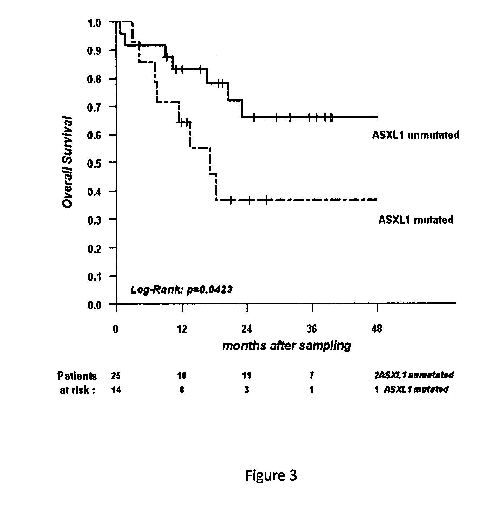

To determine whether *ASXL1* had prognostic impact independently of acute transformation, we compared the overall survival of patients mutated for *ASXL1* but who had not experienced acute progression to that of unmutated patients: *ASXL1* mutation was associated with a poor outcome (Fig 3). Finally, within MP-CMML patients, cases mutated for *ASXL1* had a poorer survival than the unmutated cases. We did the same analysis for *TET2* mutational status. In contrast to *ASXL1, TET2* had no impact on overall survival (data not shown).

### 3) Alteration of the ASXL1 gene in patients suffering from MPN

### Mutation of ASXL1in MPN

To determine whether ASXL1 could be involved in other types of myeloid diseases, we studied the ASXL1 gene in 64 myeloproliferative neoplasms (MPNs).

Our series comprised 10 cases of polycythemia vera, 35 cases of essential thrombo- cythemia (ET), 10 cases of primary myelofibrosis (PMF), 1 case of prefibrotic PMF, 5 MPNs at blast phase and 3 unclassifiable MPNs.

We also searched for mutations in 12 non-MPN cases comprising 7 secondary thrombocytosis and 5 secondary erythrocytosis. All patients signed an informed consent and the study was approved by our ethical committee.

We searched for ASXL1 mutations as described previously, and simultaneously for JAK2 (V617F) and TET2 (all exons) mutations.

The results have shown that heterozygous TET2 frameshift mutations are found in 4 out of the 64 MPN cases (6.2%), 2 ET and 2 PMF.

We also found heterozygous frameshift mutations of ASXL1 in 5 cases (7.8%) including 1 ET out of 35, 3 PMF out of 10 (1 was in accelerated phase) and 1 acute myeloid leukemia (AML) post-ET. None of the five ASXL1-mutated cases carried a JAK2 V617F mutation and only one of these five cases (a PMF) was also mutated for TET2. The four other TET2 cases did not have a TET2 mutation but two of them showed an abnormal karyotype.

We analyzed the same sequences in DNA extracted from CD34-purified cells of three patients with ASXL1 and/or TET2 mutation in their blood cell DNA (HD-0496, HD-0536 and HD- 0540). The same ASXL1 and TET2 mutations were detected in the corresponding CD34 DNA. This is in agreement with what is known of the physiopathology of MPNs and suggests that ASXL1 mutations occur early during disease evolution.

Finally, ASXL1 mutations were found in nearly 8% of patients suffering from MPNs, and more especially in 33% of patients suffering from PMF.

### Mutation of ASXL1innon-CML MPNs

The previous analysis was done for 112 new MPN cases comprising 97 PV, ET and MF, 9 blast-phase PV/ET/MF and 6 unclassified MPN and MPN/MDS forms. We also searched for mutations in 32 non-MPN cases comprising 10 reactive thrombocytosis (RT) and 22 reactive erythrocytosis (RE).

*ASXL1*mutations were found in 13 cases (11.6%). These mutations were all heterozygous and comprised 10 frameshift (including 7 c.1934dupG p.Gly646TrpfsX12) or nonsense mutations presumed to truncate the protein from its C-terminus that includes the plant homeodomain finger domain (PHD) (Table IV).

Disease-specific mutational frequencies were 8% in PV, 4% in ET, 12% in PMF, 66% in post-PV MF, 25% in post-ET MF, 22% in blast-phase PV/ET/MF and 66% in MPN/MDS. *TET2* mutations were present in 11 of 112 patients (10%). Interestingly, none of the 32 reactive cases was mutated for *ASXL1.*

Because differential diagnosis between ET (MPN) and reactive thrombocytosis may be difficult, the presence of an *ASXL1* mutation could help in the diagnosis of MPN to exclude a reactive thrombocytosis.

The presence of an *ASXL1* mutation did not influence leukocyte count, hemoglobin or hematocrit levels, but platelet count was lower in ASXL1-mutated cases (416 x 10⁹ cells/liter, p=0.009) as compared to ASXL1 wt (625 x 10⁹ cells/liter).

Finally, we observed a high incidence of *ASXL1* mutation in MF patients including PMF, post-ET MF and post-PV MF, and a low incidence in ET and PV, implying that *ASXL1* may be associated with a more aggressive phenotype. Moreover, the proportion of *ASXL1* mutations was high in post-PV MF and post-ET MF (66% and 25%, respectively); suggesting that the *ASXL1* status might be used to predict the risk of evolution of PV and ET into MF.

### 4) Alteration of the ASXL1 gene in patients suffering from AML

To determine whether ASXL1 is involved in AML, we used DNA sequencing and aCGH as described previously to search for mutations and deletions of the gene in 46 cases of AML with normal karyotype and 17 cases with trisomy 8 (n = 14), 9q deletion (HD-0632), trisomy 11 (HD-0304) or 20q11-13 deletion (HD-0381) as a sole karyotypic abnormality.

The 63 AMLs were 46 primary cases and 17 transformations of a previous myeloid disease. We did not include therapy-related AMLs. All patients signed an informed consent and the study was approved by our institutional review board.

In all, 41 out of the 50 cases studied by aCGH did not show any copy number aberration (isolated trisomy 8 was not taken into account).

We found heterozygous nonsense or frameshift mutations of ASXL1 in 11 out of the 63 cases (17.5%). We also found several cases of substitution, which we did not take into account because they may represent polymorphisms. The deletion of chromosomal region 20q11-13 (HD-0381), which was visible on the karyotype, involved ASXL1. Thus, in total, 12 cases out of 63 showed ASXL1 alteration (19%).

In two cases, we sequenced ASXL1 in DNA extracted from buccal smears of the patient with ASXL1 mutation; ASXL1 was not mutated, showing that the mutation was acquired.

In agreement with what is known for AML with normal karyotype,5 almost half of the cases (28 out of 63, 44%) showed exon 12 NPM1 mutations. NPM1 mutations were mutually exclusive with ASXL1 alterations: none of the 28 NPM1-mutated cases showed ASXL1 mutation or deletion, whereas 12 out of the 35 (34%) non-NPM1-mutated cases were mutated or deleted for ASXL1.

FLT3 mutations and internal tandem duplications (ITD) were found in 19 cases (30%) and were not observed with ASXL1 mutations except in one case (HD-0282).

Three cases were mutated in either K or NRAS, and one case in JAK2. Data on CEBPA, available for 11 cases, revealed no mutation.

Actually, the difference between NPM1 and ASXL1 mutations may rather reflect two different routes of leukemogenesis than two alternate hits on the same route. NPM1 mutations are uncommon in AMLs secondary to a chronic myeloid disease. In contrast, 9 of the 17 secondary AMLs (i.e.53%) showed ASXL1 mutation or deletion (vs 3 of 46 primary AMLs, including a case with mixed lineage dysplasia) and ASXL1 alterations were prominently observed in AMLs secondary to a chronic myeloid disease (9 of 12, 75%), whereas it was not the case for NPM1 mutations (2 of 28).

Were this hypothesis validated by the study of more cases, the detection of ASXL1 mutations would help distinguish between primary and secondary AMLs, and consequently help orient the prognosis, even in the absence of known chronic phases.

In contrast, TET2 mutations in a series of AML were found to correlate neither with the presence or absence of NPM1 or FLT3 mutations nor with an antecedent of chronic myeloid disease. 10 In another series, three of four TET2 mutations were found in secondary cases.

Although TET2 and ASXL1 may function in similar pathways of epigenetic regulation, there might be differences in their relationship with NPM1 and in their window of action during the course of the disease.

In the case that has both ASXL1 and FLT3 alteration, we determined that the ASXL1 mutation, but not the FLT3 ITD, was present at the chronic phase, suggesting that the ASXL1 and FLT3 mutations can cooperate in rare cases.

Finally, the rare RAS mutations occurred indifferently in primary or secondary cases; one was found in an ASXL1- mutated case, which was not unexpected, as we have previously shown that RAS and ASXL1 mutations can co-occur.2 It will now be interesting to determine what other alterations can associate with either the NPM1 or ASXL1 route of leukemogenesis.

### 5) frame shift p.Gly646Trpfsx12 mutation is strongly correlated with RAEB2

All patients signed an informed consent and the study was approved by our institutional review board. They include 65 cases of MDS. According to the WHO criteria, the panel comprised 5 refractory anemia (RA), 13 refractory anemia with ring sideroblasts (RARS) (including one with myelofibrosis), 7 refractory cytopenia with multilineage dysplasia (RCMD), 16 refractory anemia with excess of blasts type 1 (RAEB1), 19 refractory anemia with excess of blasts type 2 (RAEB2) and 5 MDS-unclassified (MDS-U) cases. Six cases were secondary to hematopoietic or non-hematopoietic diseases. The majority of MDS samples were collected at the time of diagnosis; some were in therapeutic abstention of a known MDS and some were under symptomatic treatment. Seventeen cases were IPPS low risk (0), 23 were int-1 (0.5-1), 12 were int-2 (1.5-2) and 7 were high risk (≥2.5).

DNA sequencing of exon-coding sequences of *ASXL1*, *CBL, FLT3, IDH1*, *IDH2, JAK2, KRAS, NPM1, NRAS, RUNX1, TET2* and *WT1* was done as follows. PCR amplifications of bone marrow cell DNA were done in a total volume of 25 µl PCR mix containing at least 5 ng template DNA, Taq buffer, 200 µmol of each deoxynucleotide triphosphate, 20 pmol of each primer and 1 unit of Hot Star Taq (Qiagen). PCR amplification conditions were as follows: 95°C 10 min; 95°C 30 sec, 55°C 30 sec, 72°C 30 sec to 1min depending on PCR product length for 35 cycles; 72°C 10 min. PCR products were purified using MILLIPORE plate MSNU030. One microliter of the purified PCR products was used for sequencing using the Big Dye terminator v1.1 kit (APPLIED BIOSYSTEMS) including the forward or reverse primer. After G50 purification, sequences were loaded on an ABI 3130XL automat (APPLIED BIOSYSTEMS). The sequence data files were analyzed using both SEQSCAPE and PHRED/PHRAP/CONSED softwares and all mutations were confirmed on an independent PCR product.

*ASXL1* exon 12 frameshift mutations (11 times the same p.Gly646Trpfsx12) were observed in 12 out of the 65 MDS cases (18.5%) including 1 out of 5 RA (20%), 2 out of 16 RAEB1 (12.5%) and 9 out of 19 RAEB2 (47.4%).

We found 12 cases with *TET2* mutation (18.5%) and 4 with *RUNX1* mutation (6.2%). One patient (HD-0311) had two *TET2* mutations. *TET2* mutations were frequent in RAEB1 (7/16, 43.8%). Mutations in *RUNX1* and *TET2* were mutually exclusive but both could associate with *ASXL1* mutations: two cases showed both an *ASXL1* and a *TET2* mutation and three cases both an *ASXL1* and a *RUNX1* mutation. One case of *ASXL1* deletion (HD-0190) and one case of *TET2* deletion (HD-0145) have been reported. One case (HD-0232) had a break in *RUNX1* detected by aCGH (not shown).

We did not find any *FLT3, NPM1* or *WT1* mutation. One MDS-U had a *JAK2* mutation and one RCMD case a *KRAS* mutation. Five cases, all RAEB2, were mutated in *CBL.* In one of these the mutation was homozygous. One subtitution occurred in the case with trisomy 11 (HD-0264), and showed a 2/3 ratio with the wild-type residue, suggesting that the mutated allele was duplicated.

We found 5 IDH mutations in the 65 cases (7.7%), including 2 mutations in *IDH1* and 3 in *IDH2.*

Based on the known functions of the proteins, on a previous model and classification on where the mutations were present (MDSs and/or secondary AMLs and/or primary AMLs) and on how they combined, we tentatively grouped the genes in four classes.

The first class (we called "initiators") includes *RUNX1* and *TET2.* They may cause clonal dominance of hematopoietic stem cells.

*ASXL1* and *NPM1* would constitute class II ("selectors"). Mutations in these genes may select a leukemogenic pathway leading towards either primary or secondary AML.

Genes associated with proliferation (*CBL, FLT3, JAK2, RAS)* define class III ("amplifiers"). *JAK2* mutation plays little role in MDSs and NK-AMLs.

Finally, for three reasons we grouped *IDH1, IDH2,* and *WT1* in a putative class IV we provisionally called "boosters". First, *IDH* and *WT1* mutations were exclusive but could co-occur with mutations in genes from other classes. Second, they occurred primarily in AMLs and were rare in MDSs (and also in myeloproliferative neoplasms). Third, mutations of these genes could be associated with modifications of the HIF1 and oxygen-sensing pathways. Class IV mutations are rather associated with acute phase.

Overall, AML cases had zero, one or three mutations. Because of this, and although no case had four mutations, we propose that AML develops following - at least - four cooperating mutations, one from each class. This is speculative and the identification of new target genes and the study of others will lead to a more precise picture.

## Claims

1. A method for the prognosis of the progression of a polycythemia vera (PV) to a post-polycythemia vera myelofibrosis (post-PV MF) in a subject, said method comprising the step of analyzing a biological sample from said subject suffering from a PV by determining the presence or the absence of a mutation in the *ASXL1* (additional sex combs like 1) gene coding for the polypeptide having the sequence SEQ ID N°2, wherein the presence of an *ASXL1* mutation is indicative of a risk of a progression of said PV to Post-PV MF.

2. A method for the prognosis of the progression of an essential thrombocythemia (ET) to a post-essential thrombocythemia myelofibrosis (post-ET MF) in a subject, said method comprising the step of analyzing a biological sample from said subject suffering from an ET by determining the presence or the absence of a mutation in the *ASXL1* (additional sex combs like 1) gene coding for the polypeptide having the sequence SEQ ID N°2, wherein the presence of an ASXL1 mutation is indicative of a risk of a progression of said ET to Post-ET MF.

3. The method of claim 1 or 2, wherein said subject is a human.

4. The method according to any one of claims 1 to 3, wherein said biological sample is a bone marrow sample.

5. The method according to any one of claims 1 to 4, wherein said mutation is selected from insertions, deletions, and point mutations corresponding to missense mutations and nonsense mutations.

6. The method according to any one of claims 1 to 5, wherein said mutation results in the expression of a mutated *ASXL1* protein, said mutated *ASXL1* protein does not comprise any PHD domain (SEQ ID N°3) or a fragment thereof.

7. The method according to claim 6, wherein said mutation is a non sense mutation leading to the introduction of a stop codon (X) in the open reading frame of the *ASXL1* protein and is preferably selected in the group comprising Tyr591X, Gln592X, Lys618X, Arg693X, Gln759X, Gln768X, Leu775X and Arg1068X.

8. The method according to claim 6, wherein said mutated protein can result from a frame shift (FS) because of an insertion or a deletion in the *ASXL1* gene and said mutation is preferably selected in the group comprising Gly64Trp FS, Arg596Pro FS, Ala611Arg FS, His630Pro FS, Gly646Trp FS, Leu762Phe FS, Trp796Gly FS, Thr822Asn FS, Thr836Leu FS, Ser846Gln FS, Asp879Glu FS, Lys888Glu FS, Leu1213Ile FS, Pro1263Gln FS, Leu1266His FS, Trp1271Lys FS, or Ser1457Pro FS.

9. The method according to any one of claims 1 to 8, wherein the determining step is carried out on genomic DNA or on *ASXL1* mRNA/cDNA.

10. The method according to any one of claims 1 to 8, wherein the determining step is carried out on *ASXL1* protein.

## Patentansprüche

1. Ein Verfahren für die Prognose der Progression einer Polycythaemia vera (PV) zu einer Post-Polycythaemia vera Myelofibrose (Post-PV MF) in einem Subjekt, das Verfahren umfassend den Schritt des Analysierens einer biologischen Probe von dem Subjekt, das an einer PV leidet durch Bestimmen der Anwesenheit oder der Abwesenheit einer Mutation im *ASXL1* (additional sex combs like 1) Gen kodierend für das Polypeptid, das die SEQ ID NO:2 hat, wobei die Anwesenheit einer *ASXL1*-Mutation für ein Risiko einer Progression der PV zu einer Post-PV MF hinweisend ist.

2. Ein Verfahren für die Prognose der Progression einer essenziellen Thrombozythämie (ET) zu einer post-essentiellen Thrombozythämie Myelofibrose (Post-ET MF) in einem Subjekt, das Verfahren umfassend des Schritt des Analysierens einer biologischen Probe von dem Subjekt, das an einer ET leidet, durch Bestimmen der Anwesenheit oder der Abwesenheit einer Mutation im *ASXL1* (additional sex combs like 1) Gen kodierend für das Polypeptid, das die SEQ ID NO:2 hat, wobei die Anwesenheit einer ASXL1-Mutation für ein Risiko einer Progression der ET zu einer Post-ET MF hinweisend ist.

3. Das Verfahren nach Anspruch 1 oder 2, wobei das Subjekt ein Mensch ist.

4. Das Verfahren nach einem der Ansprüche 1 bis 3, wobei die biologische Probe eine Knochenmarksprobe ist.

5. Das Verfahren nach einem der Ansprüche 1 bis 4, wobei die Mutation ausgewählt ist aus Insertionen, Deletionen und Punktmutationen, die Missense-Mutationen und Nonsense-Mutationen entsprechen.

6. Das Verfahren nach einem der Ansprüche 1 bis 5, wobei die Mutation zur Expression eines mutierten *ASXL1*-Proteins führt, wobei das mutierte *ASXL1*-Protein nicht eine PHD-Domäne (SEQ ID NO:3) oder ein Fragment davon umfasst.

7. Das Verfahren nach Anspruch 6, wobei die Mutation eine Nonsense-Mutation ist, die zur Einführung eines Stopcodons (X) im offenen Leseraster des *ASXL1*-Proteins führt und bevorzugt ausgewählt ist aus der Gruppe umfassend Tyr591X, Gln592X, Lys618X, Arg693X, Gln759X, Gln768X, Leu775X und Arg1068X.

8. Das Verfahren nach Anspruch 6, wobei das mutierte Protein aus einer Leserasterverschiebung (FS) resultieren kann aufgrund einer Insertion oder einer Deletion im *ASXL1*-Gen und die Mutation bevorzugt ausgewählt ist aus der Gruppe umfassend Gly64Trp FS, Arg596Pro FS, Ala611Arg FS, His630Pro FS, Gly646Trp FS, Leu762Phe FS, Trp796Gly FS, Thr822Asn FS, Thr836Leu FS, Ser846Gln FS, Asp879Glu FS, Lys888Glu FS, Leu1213Ile FS, Pro1263Gln FS, Leu1266His FS, Trp1271Lys FS oder Ser1457Pro FS.

9. Das Verfahren nach einem der Ansprüche 1 bis 8, wobei der Bestimmungsschritt an genomischer DNA oder an *ASXL1*-mRNA/cDNA durchgeführt wird.

10. Das Verfahren nach einem der Ansprüche 1 bis 8, wobei der Bestimmungsschritt an *ASXL1-*Protein durchgeführt wird.

## Revendications

1. Méthode pour le pronostic de la progression d'une polycythémie vraie (PV) vers une myélofibrose post-polycythémie vraie (MF post-PV) chez un sujet, ladite méthode comprenant l'étape d'analyse d'un échantillon biologique provenant dudit sujet souffrant d'une PV par la détermination de la présence ou de l'absence d'une mutation dans le gène *ASXL1* (additional sex combs like 1) codant pour le polypeptide ayant la séquence SEQ ID NO : 2, dans laquelle la présence d'une mutation d'*ASXL1* indique un risque de progression de ladite PV vers une MF post-PV.

2. Méthode pour le pronostic de la progression d'une thrombocythémie essentielle (TE) vers une myélofibrose post-thrombocythémie essentielle (MF post-TE) chez un sujet, ladite méthode comprenant l'étape d'analyse d'un échantillon provenant dudit sujet souffrant d'une TE par la détermination de la présence ou de l'absence d'une mutation dans le gène *ASXL1* (additional sex combs like 1) codant pour le polypeptide ayant la séquence SEQ ID NO : 2, dans laquelle la présence d'une mutation d'*ASXL1* indique un risque de progression de ladite TE vers une MF post-TE.

3. Méthode selon la revendication 1 ou 2, dans laquelle ledit sujet est un être humain.

4. Méthode selon l'une quelconque des revendications 1 à 3, dans laquelle ledit échantillon biologique est un échantillon de moelle osseuse.

5. Méthode selon l'une quelconque des revendications 1 à 4, dans laquelle ladite mutation est choisie parmi des insertions, des délétions, et des mutations ponctuelles correspondant à des mutations faux-sens et à des mutations non-sens.

6. Méthode selon l'une quelconque des revendications 1 à 5, dans laquelle ladite mutation entraîne l'expression d'une protéine *ASXL1* mutée, ladite protéine *ASXL1* mutée ne comprend aucun domaine PHD (SEQ ID NO : 3) ni l'un de ses fragments.

7. Méthode selon la revendication 6, dans laquelle ladite mutation est une mutation non-sens menant à l'introduction d'un codon d'arrêt (X) dans le cadre de lecture ouvert de la protéine *ASXL1* et est choisie de préférence dans le groupe comprenant Tyr591X, Gln592X, Lys618X, Arg693X, Gln759X, Gln768X, Leu775X et Arg1068X.

8. Méthode selon la revendication 6, dans laquelle ladite protéine mutée peut résulter d'un décalage du cadre (FS) à cause d'une insertion ou d'une délétion dans le gène *ASXL1* et ladite mutation est choisie de préférence dans le groupe comprenant Gly64Trp FS, Arg596Pro FS, Ala611Arg FS, His630Pro FS, Gly646Trp FS, Leu762Phe FS, Trp796Gly FS, Thr822Asn FS, Thr836Leu FS, Ser846Gln FS, Asp879Glu FS, Lys888Glu FS, Leu1213Ile FS, Pro1263Gln FS, Leu1266His FS, Trp1271Lys FS, ou Ser1457Pro FS.

9. Méthode selon l'une quelconque des revendications 1 à 8, dans laquelle l'étape de détermination est réalisée sur l'ADN génomique ou sur l'ARNm/ADNc de *ASXL1.*

10. Méthode selon l'une quelconque des revendications 1 à 8, dans laquelle l'étape de détermination est réalisée sur la protéine *ASXL1.*


### SEQUENCE LISTING

<110> GELSI-BOYER, Véronique BIRNBAUM, Daniel
<120> ASXL1 AS A NEW DIAGNOSTIC MARKER OF MYELOID NEOPLASMS
<130> IPS-B-0001 PCT
<150> us 61/303,971
   <151> 2010-02-12
<160> 33
<170> PatentIn version 3.5
<210> 1
   <211> 7056
   <212> DNA
   <213> Homo sapiens
<400> 1
   cacacccacg gcagacacgc acgcacccgg gcgccgaagg gaaagccgcg tctcgccctc      60
   ccgccccgcc gtcggtcctg tctcagtccc tcagcagagc gggaaagcgg aggccggagc     120
   cgtgacctct gaccccgtgg ttatgcggag ccgccgcatt ccttagcgat cgcggggcag     180
   ccgccgctgc cgccgtgggc gactgacgca gcgcgggcgc gtggagccgc cgccgcccct     240
   cccccaccgc cgctctcgcg ccagccggtc cccgcgtgcc cgccccttct ccccggccgc     300
   acccgagacc tcgcgcgccg ccgctgccac gcgccccccc caccgccgcc gccgccccag     360
   ccccgcgcca ccgccccagc ccgcccagcc cggaggtccc gcgtggagct gccgccgccg     420
   ccggggagaa ggatgaagga caaacagaag aagaagaagg agcgcacgtg ggccgaggcc     480
   gcgcgcctgg tattagaaaa ctactcggat gctccaatga caccaaaaca gattctgcag     540
   gtcatagagg cagaaggact aaaggaaatg agaagtggga cttcccctct cgcatgcctc     600
   aatgctatgc tacattccaa ttcaagagga ggagaggggt tgttttataa actgcctggc     660
   cgaatcagcc ttttcacgct caagaaggat gccctgcagt ggtctcgcca tccagctaca     720
   gtggagggag aggagccaga ggacacggct gatgtggaga gctgtgggtc taatgaagcc     780
   agcactgtga gtggtgaaaa cgatgtatct cttgatgaaa catcttcgaa cgcatcctgt     840
   tctacagaat ctcagagtcg acctctttcc aatcccaggg acagctacag agcttcctca     900
   caggcgaaca aacaaaagaa aaagactggg gtgatgctgc ctcgagttgt cctgactcct     960
   ctgaaggtaa acggggccca cgtggaatct gcatcagggt tctcgggctg ccacgccgat    1020
   ggcgagagcg gcagcccgtc cagcagcagc agcggctctc tggccctggg cagcgctgct    1080
   attcgtggcc aggccgaggt cacccaggac cctgccccgc tcctgagagg cttccggaag    1140
   ccagccacag gtcaaatgaa gcgcaacaga ggggaagaaa tagattttga gacacctggg    1200
   tccattcttg tcaacaccaa cctccgtgcc ctgatcaact ctcggacctt ccatgcctta    1260
   ccatcacact tccagcagca gctcctcttc ctcctgcctg aagtagacag acaggtgggg    1320
   acggatggcc tgttgcgtct cagcagcagt gcactaaata acgagttttt tacccatgcg    1380
   gctcagagct ggcgggagcg cctggctgat ggtgaattta ctcatgagat gcaagtcagg 1440
   atacgacagg aaatggagaa ggaaaagaag gtggaacaat ggaaagaaaa gttctttgaa 1500
   gactactatg gacagaagct gggtttgacc aaagaagagt cattgcagca gaacgtgggc 1560
   caggaggagg ctgaaatcaa aagtggcttg tgtgtcccag gagaatcagt gcgtatacag 1620
   cgtggtccag ccacccgaca gcgagatggg cattttaaga aacgctctcg gccagatctc 1680
   cgaaccagag ccagaaggaa tctgtacaaa aaacaggagt cagaacaagc aggggttgct 1740
   aaggatgcaa aatctgtggc ctcagatgtt cccctctaca aggatgggga ggctaagact 1800
   gacccagcag ggctgagcag tccccatctg ccaggcacat cctctgcagc acccgacctg 1860
   gagggtcccg aattcccagt tgagtctgtg gcttctcgga tccaggctga gccagacaac 1920
   ttggcacgtg cctctgcatc tccagacaga attcctagcc tgcctcagga aactgtggat 1980
   caggaaccca aggatcagaa gaggaaatcc tttgagcagg cggcctctgc atcctttccc 2040
   gaaaagaagc cccggcttga agatcgtcag tcctttcgta acacaattga aagtgttcac 2100
   accgaaaagc cacagcccac taaagaggag cccaaagtcc cgcccatccg gattcaactt 2160
   tcacgtatca aaccaccctg ggtggttaaa ggtcagccca cttaccagat atgcccccgg 2220
   atcatcccca ccacggagtc ctcctgccgg ggttggactg gcgccaggac cctcgcagac 2280
   attaaagccc gtgctctgca ggtccgaggg gcgagaggtc accactgcca tagagaggcg 2340
   gccaccactg ccatcggagg ggggggtggc ccgggtggag gtggcggcgg ggccaccgat 2400
   gagggaggtg gcagaggcag cagcagtggt gatggtggtg aggcctgtgg ccaccctgag 2460
   cccaggggag gcccgagcac ccctggaaag tgtacgtcag atctacagcg aacacaacta 2520
   ctgccgcctt atcctctaaa tggggagcat acccaggccg gaactgccat gtccagagct 2580
   aggagagagg acctgccttc tctgagaaag gaggaaagct gcctactaca gagggctaca 2640
   gttggactca cagatgggct aggagatgcc tcccaactcc ccgttgctcc cactggggac 2700
   cagccatgcc aggccttgcc cctactgtcc tcccaaacct cagtagctga gagattagtg 2760
   gagcagcctc agttgcatcc ggatgttaga actgaatgtg agtctggcac cacttcctgg 2820
   gaaagtgatg atgaggagca aggacccacc gttcctgcag acaatggtcc cattccgtct 2880
   ctagtgggag atgatacatt agagaaagga actggccaag ctcttgacag tcatcccact 2940
   atgaaggatc ctgtaaatgt gacccccagt tccacacctg aatcctcacc gactgattgc 3000
   ctgcagaaca gagcatttga tgacgaatta gggcttggtg gctcatgccc tcctatgagg 3060
   gaaagtgata ctagacaaga aaacttgaaa accaaggctc tcgtttctaa cagttctttg 3120
   cattggatac ccatcccatc gaatgatgag gtagtgaaac agcccaaacc agaatccaga 3180
   gaacacatac catctgttga gccccaggtt ggagaggagt gggagaaagc tgctcccacc 3240
   cctcctgcat tgcctgggga tttgacagct gaggagggtc tagatcctct tgacagcctt 3300
   acttcactct ggactgtgcc atctcgagga ggcagtgaca gcaatggcag ttactgtcaa 3360
   caggtggaca ttgaaaagct gaaaatcaac ggagactctg aagcactgag tcctcacggt 3420
   gagtccacgg atacagcctc tgactttgaa ggtcacctca cggaggacag cagtgaggct     3480
   gacactagag aagctgcagt gacaaaggga tcttcggtgg acaaggatga gaaacccaat     3540
   tggaaccaat ctgccccact gtccaaggtg aatggtgaca tgcgtctggt tacaaggaca     3600
   gatgggatgg ttgctcctca gagctgggtg tctcgagtat gtgcggtccg ccaaaagatc     3660
   ccagattccc tactgctggc cagtactgag taccagccaa gagccgtgtg cctgtccatg     3720
   cctgggtcct cagtggaggc cactaaccca cttgtgatgc agttgctgca gggtagcttg     3780
   cccctagaga aggttcttcc accagcccac gatgacagca tgtcagaatc cccacaagta     3840
   ccacttacaa aagaccagag ccatggctcg ctacgcatgg gatctttaca tggtcttgga     3900
   aaaaacagtg gcatggttga tggaagcagc cccagttctt taagggcttt gaaggagcct     3960
   cttctgccag atagctgtga aacaggcact ggtcttgcca ggattgaggc cacccaggct     4020
   cctggagcac cccaaaagaa ttgcaaggca gtcccaagtt ttgactccct ccatccagtg     4080
   acaaatccca ttacatcctc taggaaactg gaagaaatgg attccaaaga gcagttctct     4140
   tcctttagtt gtgaagatca gaaggaagtc cgtgctatgt cacaggacag taattcaaat     4200
   gctgctccag gaaagagccc aggagatctt actacctcga gaacacctcg tttctcatct     4260
   ccaaatgtga tctcctttgg tccagagcag acaggtcggg ccctgggtga tcagagcaat     4320
   gttacaggcc aagggaagaa gctttttggc tctgggaatg tggctgcaac ccttcagcgc     4380
   cccaggcctg cggacccgat gcctcttcct gctgagatcc ctccagtttt tcccagtggg     4440
   aagttgggac caagcacaaa ctccatgtct ggtggggtac agactccaag ggaagactgg     4500
   gctccaaagc cacatgcctt tgttggcagc gtcaagaatg agaagacttt tgtggggggt     4560
   cctcttaagg caaatgccga gaacaggaaa gctactgggc atagtcccct ggaactggtg     4620
   ggtcacttgg aagggatgcc ctttgtcatg gacttgccct tctggaaatt accccgagag     4680
   ccagggaagg ggctcagtga gcctctggag ccttcttctc tcccctccca actcagcatc     4740
   aagcaggcat tttatgggaa gctttctaaa ctccaactga gttccaccag ctttaattat     4800
   tcctctagct ctcccacctt tcccaaaggc cttgctggaa gtgtggtgca gctgagccac     4860
   aaagcaaact ttggtgcgag ccacagtgca tcactttcct tgcaaatgtt cactgacagc     4920
   agcacggtgg aaagcatctc gctccagtgt gcgtgcagcc tgaaagccat gatcatgtgc     4980
   caaggctgcg gtgcgttctg tcacgatgac tgtattggac cctcaaagct ctgtgtattg     5040
   tgccttgtgg tgagataata aattatggcc atgggaaaca ttgtatattt agtgtgtgta     5100
   ttttgataat gattgatctt aaatctgtat acagaatatc attgatataa tactctttag     5160
   gcaggagcac tcttgccttc ccccaaaatt tacactgcta aagccctctg tcacttggcg     5220
   acccttctgg tcttgctgga ggggtttcct gggtataacc cattgggctg cccaaggcca     5280
   gccagcctga gctctcctgc aagacagagc ctgatgtggc acggagtggg gttgcggggg     5340
   gtggggggac tgcctgactc ccagagggac ttgaaactga agcaagaagg ttgcattctc     5400
   caccaaggga gttaacctac ctgaactaag tagaaatgcc agtcttccac taccccctcc     5460
   ctgccatctt ttcttctgct actttgggga gttgatggcc aggaaagaag ccagcacagg 5520
   gttaaagtaa ctcctggcat tgcccaccag ggggctggtg cacctgctga cctcagggtc 5580
   acagttgagt catttgccag ttgacggagc aagtttgacc ttggttctgt tgctgaagca 5640
   aatttggaac ttttctgtct cagtgtgatc cactaaccca caggatcatt tggaaccttg 5700
   aatagctctg cttggacaat ggggttgggg aatagggttg tctttcctat gaaaatgcca 5760
   tctgtagacc ttgtgagtca gccgtccaga tgtttgcagg tgaattcctc tgcttgacat 5820
   cctccctgtc actttggacc ctatgggagt gggcatctcc acgcacctgt gtatgtgaaa 5880
   gtcattttac atttcaaagc agtgtgtgtt tcttattttt atatttttaa ctctttattc 5940
   ttggatgtat aaagtgaact ttttggcttc tgtaagtatg ctctatgcac ctctaatgtt 6000
   ttatcatgta tttatatgtt gtacacagta ctggctgatt ctgtaaatgg atgtattgta 6060
   cagagaacat gaacgtctct tcctaatttt acatcttcag catcattgca ttaaagtggt 6120
   gtaatctcct tctctacatc tgttgtcaga gccactgagt gctgtgctgc tcgacgtgag 6180
   ggtgaaatga ttgacttgtg acctgccagg ttgcccgatg ccctgttggg tcaccggctg 6240
   gacctgctgc agcctgcaga gccacagtca gcctgcccac atgccaccga gcaaacgcat 6300
   cttgcttttc acatctctcc tcctacagcc ttaatggctg cttgctgcca tatgtgacaa 6360
   atcaccacca ccagtgttaa gtgcttctgg attcatgggt gagttccctg ggcagccccc 6420
   aggaaggcct tccagatctg gctccagggt caccacctgt cacagcaata cctgggacca 6480
   tgctctcctg ggactgtgag gctccttttg acgtactttt gacatcaggc aggtttggga 6540
   agaaacaaag ccatgcctgc tcctgcctct ctcccaacat gtttccagca agtagatgcc 6600
   cctgtgtgtg ttttcccttg ccttgtttcc tgccttatat cttgtatttc gacttattac 6660
   agagttgagg gttcttgctt aatttagatc aagtataaaa tttgtatgac ttcaagtctc 6720
   attttatctg aaaggttttt ttctcattta atctgatgtg gcattttcgt catctgaagc 6780
   atgagtgaca agttgggaat gatgtggtga tttagaatgc agtattggcc aagtccaagt 6840
   tgtcaactta agcgtctgtt taccaaagac cgggaacagg ggcccaaaca tgtccagtcc 6900
   tcttcttccc tctgctggaa cctttgggga cactcaaggg tacagtttga cactgatctg 6960
   gtccatgagg ctgcccagag aaagcactgc ttctgtatgt ctcttgtggt attggaacaa 7020
   taaacccgta caacctgcaa aaaaaaaaaa aaaaaa 7056
<210> 2
   <211> 1541
   <212> PRT
   <213> Homo sapiens
<400> 2
   Met Lys Asp Lys Gln Lys Lys Lys Lys Glu Arg Thr Trp Ala Glu Ala
   1               5                   10                  15
   Ala Arg Leu Val Leu Glu Asn Tyr Ser Asp Ala Pro Met Thr Pro Lys
               20                  25                  30
   Gln Ile Leu Gln Val Ile Glu Ala Glu Gly Leu Lys Glu Met Arg Ser
           35                  40                  45
   Gly Thr Ser Pro Leu Ala Cys Leu Asn Ala Met Leu His Ser Asn Ser
       50                  55                  60
   Arg Gly Gly Glu Gly Leu Phe Tyr Lys Leu Pro Gly Arg Ile Ser Leu
   65                  70                  75                  80
   Phe Thr Leu Lys Lys Asp Ala Leu Gln Trp Ser Arg His Pro Ala Thr
                   85                  90                  95
   Val Glu Gly Glu Glu Pro Glu Asp Thr Ala Asp Val Glu Ser Cys Gly
               100                 105                 110
   Ser Asn Glu Ala Ser Thr Val Ser Gly Glu Asn Asp Val Ser Leu Asp
           115                 120                 125
   Glu Thr Ser Ser Asn Ala Ser Cys Ser Thr Glu Ser Gln Ser Arg Pro
       130                 135                 140
   Leu Ser Asn Pro Arg Asp Ser Tyr Arg Ala Ser Ser Gln Ala Asn Lys
   145                 150                 155                 160
   Gln Lys Lys Lys Thr Gly Val Met Leu Pro Arg Val Val Leu Thr Pro
                   165                 170                 175
   Leu Lys Val Asn Gly Ala His Val Glu Ser Ala Ser Gly Phe Ser Gly
               180                 185                 190
   Cys His Ala Asp Gly Glu Ser Gly Ser Pro Ser Ser Ser Ser Ser Gly
           195                 200                 205
   Ser Leu Ala Leu Gly Ser Ala Ala Ile Arg Gly Gln Ala Glu Val Thr
       210                 215                 220
   Gln Asp Pro Ala Pro Leu Leu Arg Gly Phe Arg Lys Pro Ala Thr Gly
   225                 230                 235                 240
   Gln Met Lys Arg Asn Arg Gly Glu Glu Ile Asp Phe Glu Thr Pro Gly
                   245                 250                 255
   Ser Ile Leu Val Asn Thr Asn Leu Arg Ala Leu Ile Asn Ser Arg Thr
               260                 265                 270
   Phe His Ala Leu Pro Ser His Phe Gln Gln Gln Leu Leu Phe Leu Leu
           275                 280                 285
   Pro Glu Val Asp Arg Gln Val Gly Thr Asp Gly Leu Leu Arg Leu Ser
       290                 295                 300
   Ser Ser Ala Leu Asn Asn Glu Phe Phe Thr His Ala Ala Gln Ser Trp
   305 310 315 320
   Arg Glu Arg Leu Ala Asp Gly Glu Phe Thr His Glu Met Gln Val Arg
   325 330 335
   Ile Arg Gln Glu Met Glu Lys Glu Lys Lys Val Glu Gln Trp Lys Glu
   340 345 350
   Lys Phe Phe Glu Asp Tyr Tyr Gly Gln Lys Leu Gly Leu Thr Lys Glu
   355 360 365
   Glu Ser Leu Gln Gln Asn Val Gly Gln Glu Glu Ala Glu Ile Lys Ser
   370 375 380
   Gly Leu Cys Val Pro Gly Glu Ser Val Arg Ile Gln Arg Gly Pro Ala
   385 390 395 400
   Thr Arg Gln Arg Asp Gly His Phe Lys Lys Arg Ser Arg Pro Asp Leu
   405 410 415
   Arg Thr Arg Ala Arg Arg Asn Leu Tyr Lys Lys Gln Glu Ser Glu Gln
   420 425 430
   Ala Gly Val Ala Lys Asp Ala Lys Ser Val Ala Ser Asp Val Pro Leu
   435 440 445
   Tyr Lys Asp Gly Glu Ala Lys Thr Asp Pro Ala Gly Leu Ser Ser Pro
   450 455 460
   His Leu Pro Gly Thr Ser Ser Ala Ala Pro Asp Leu Glu Gly Pro Glu
   465 470 475 480
   Phe Pro Val Glu Ser Val Ala Ser Arg Ile Gln Ala Glu Pro Asp Asn
   485 490 495
   Leu Ala Arg Ala Ser Ala Ser Pro Asp Arg Ile Pro Ser Leu Pro Gln
   500 505 510
   Glu Thr Val Asp Gln Glu Pro Lys Asp Gln Lys Arg Lys Ser Phe Glu
   515 520 525
   Gln Ala Ala Ser Ala Ser Phe Pro Glu Lys Lys Pro Arg Leu Glu Asp
   530 535 540
   Arg Gln Ser Phe Arg Asn Thr Ile Glu Ser Val His Thr Glu Lys Pro
   545 550 555 560
   Gln Pro Thr Lys Glu Glu Pro Lys Val Pro Pro Ile Arg Ile Gln Leu
   565 570 575
   Ser Arg Ile Lys Pro Pro Trp Val Val Lys Gly Gln Pro Thr Tyr Gln
               580                 585                 590
   Ile Cys Pro Arg Ile Ile Pro Thr Thr Glu Ser Ser Cys Arg Gly Trp
           595                 600                 605
   Thr Gly Ala Arg Thr Leu Ala Asp Ile Lys Ala Arg Ala Leu Gln Val
       610                 615                 620
   Arg Gly Ala Arg Gly His His Cys His Arg Glu Ala Ala Thr Thr Ala
   625                 630                 635                 640
   Ile Gly Gly Gly Gly Gly Pro Gly Gly Gly Gly Gly Gly Ala Thr Asp
                   645                 650                 655
   Glu Gly Gly Gly Arg Gly Ser Ser Ser Gly Asp Gly Gly Glu Ala Cys
               660                 665                 670
   Gly His Pro Glu Pro Arg Gly Gly Pro Ser Thr Pro Gly Lys Cys Thr
           675                 680                 685
   Ser Asp Leu Gln Arg Thr Gln Leu Leu Pro Pro Tyr Pro Leu Asn Gly
       690                 695                 700
   Glu His Thr Gln Ala Gly Thr Ala Met Ser Arg Ala Arg Arg Glu Asp
   705                 710                 715                 720
   Leu Pro Ser Leu Arg Lys Glu Glu Ser Cys Leu Leu Gln Arg Ala Thr
                   725                 730                 735
   Val Gly Leu Thr Asp Gly Leu Gly Asp Ala Ser Gln Leu Pro Val Ala
               740                 745                 750
   Pro Thr Gly Asp Gln Pro Cys Gln Ala Leu Pro Leu Leu Ser Ser Gln
           755                 760                 765
   Thr Ser Val Ala Glu Arg Leu Val Glu Gln Pro Gln Leu His Pro Asp
       770                 775                 780
   Val Arg Thr Glu Cys Glu Ser Gly Thr Thr Ser Trp Glu Ser Asp Asp
   785                 790                 795                 800
   Glu Glu Gln Gly Pro Thr Val Pro Ala Asp Asn Gly Pro Ile Pro Ser
                   805                 810                 815
   Leu Val Gly Asp Asp Thr Leu Glu Lys Gly Thr Gly Gln Ala Leu Asp
               820                 825                 830
   Ser His Pro Thr Met Lys Asp Pro Val Asn Val Thr Pro Ser Ser Thr
           835                 840                 845
   Pro Glu Ser Ser Pro Thr Asp Cys Leu Gln Asn Arg Ala Phe Asp Asp
   850 855 860
   Glu Leu Gly Leu Gly Gly Ser Cys Pro Pro Met Arg Glu Ser Asp Thr
   865 870 875 880
   Arg Gln Glu Asn Leu Lys Thr Lys Ala Leu Val Ser Asn Ser Ser Leu
   885 890 895
   His Trp Ile Pro Ile Pro Ser Asn Asp Glu Val Val Lys Gln Pro Lys
   900 905 910
   Pro Glu Ser Arg Glu His Ile Pro Ser Val Glu Pro Gln Val Gly Glu
   915 920 925
   Glu Trp Glu Lys Ala Ala Pro Thr Pro Pro Ala Leu Pro Gly Asp Leu
   930 935 940
   Thr Ala Glu Glu Gly Leu Asp Pro Leu Asp Ser Leu Thr Ser Leu Trp
   945 950 955 960
   Thr Val Pro Ser Arg Gly Gly Ser Asp Ser Asn Gly Ser Tyr Cys Gln
   965 970 975
   Gln Val Asp Ile Glu Lys Leu Lys Ile Asn Gly Asp Ser Glu Ala Leu
   980 985 990
   Ser Pro His Gly Glu Ser Thr Asp Thr Ala Ser Asp Phe Glu Gly His
   995 1000 1005
   Leu Thr Glu Asp Ser Ser Glu Ala Asp Thr Arg Glu Ala Ala Val
   1010 1015 1020
   Thr Lys Gly Ser Ser Val Asp Lys Asp Glu Lys Pro Asn Trp Asn
   1025 1030 1035
   Gln Ser Ala Pro Leu Ser Lys Val Asn Gly Asp Met Arg Leu Val
   1040 1045 1050
   Thr Arg Thr Asp Gly Met Val Ala Pro Gln Ser Trp Val Ser Arg
   1055 1060 1065
   Val Cys Ala Val Arg Gln Lys Ile Pro Asp Ser Leu Leu Leu Ala
   1070 1075 1080
   Ser Thr Glu Tyr Gln Pro Arg Ala Val Cys Leu Ser Met Pro Gly
   1085 1090 1095
   Ser Ser Val Glu Ala Thr Asn Pro Leu Val Met Gln Leu Leu Gln
   1100 1105 1110
   Gly Ser Leu Pro Leu Glu Lys Val Leu Pro Pro Ala His Asp Asp
       1115                    1120                1125
   Ser Met Ser Glu Ser Pro Gln Val Pro Leu Thr Lys Asp Gln Ser
       1130                    1135                1140
   His Gly Ser Leu Arg Met Gly Ser Leu His Gly Leu Gly Lys Asn
       1145                    1150                1155
   Ser Gly Met Val Asp Gly Ser Ser Pro Ser Ser Leu Arg Ala Leu
       1160                    1165                1170
   Lys Glu Pro Leu Leu Pro Asp Ser Cys Glu Thr Gly Thr Gly Leu
       1175                    1180                1185
   Ala Arg Ile Glu Ala Thr Gln Ala Pro Gly Ala Pro Gln Lys Asn
       1190                    1195                1200
   Cys Lys Ala Val Pro Ser Phe Asp Ser Leu His Pro Val Thr Asn
       1205                    1210                1215
   Pro Ile Thr Ser Ser Arg Lys Leu Glu Glu Met Asp Ser Lys Glu
       1220                    1225                1230
   Gln Phe Ser Ser Phe Ser Cys Glu Asp Gln Lys Glu Val Arg Ala
       1235                    1240                1245
   Met Ser Gln Asp Ser Asn Ser Asn Ala Ala Pro Gly Lys Ser Pro
       1250                    1255                1260
   Gly Asp Leu Thr Thr Ser Arg Thr Pro Arg Phe Ser Ser Pro Asn
       1265                    1270                1275
   Val Ile Ser Phe Gly Pro Glu Gln Thr Gly Arg Ala Leu Gly Asp
       1280                    1285                1290
   Gln Ser Asn Val Thr Gly Gln Gly Lys Lys Leu Phe Gly Ser Gly
       1295                    1300                1305
   Asn Val Ala Ala Thr Leu Gln Arg Pro Arg Pro Ala Asp Pro Met
       1310                    1315                1320
   Pro Leu Pro Ala Glu Ile Pro Pro Val Phe Pro Ser Gly Lys Leu
       1325                    1330                1335
   Gly Pro Ser Thr Asn Ser Met Ser Gly Gly Val Gln Thr Pro Arg
       1340                    1345                1350
   Glu Asp Trp Ala Pro Lys Pro His Ala Phe Val Gly Ser Val Lys
       1355                    1360                1365
   Asn Glu Lys Thr Phe Val Gly Gly Pro Leu Lys Ala Asn Ala Glu
   1370 1375 1380
   Asn Arg Lys Ala Thr Gly His Ser Pro Leu Glu Leu Val Gly His
   1385 1390 1395
   Leu Glu Gly Met Pro Phe Val Met Asp Leu Pro Phe Trp Lys Leu
   1400 1405 1410
   Pro Arg Glu Pro Gly Lys Gly Leu Ser Glu Pro Leu Glu Pro Ser
   1415 1420 1425
   Ser Leu Pro Ser Gln Leu Ser Ile Lys Gln Ala Phe Tyr Gly Lys
   1430 1435 1440
   Leu Ser Lys Leu Gln Leu Ser Ser Thr Ser Phe Asn Tyr Ser Ser
   1445 1450 1455
   Ser Ser Pro Thr Phe Pro Lys Gly Leu Ala Gly Ser Val Val Gln
   1460 1465 1470
   Leu Ser His Lys Ala Asn Phe Gly Ala Ser His Ser Ala Ser Leu
   1475 1480 1485
   Ser Leu Gln Met Phe Thr Asp Ser Ser Thr Val Glu Ser Ile Ser
   1490 1495 1500
   Leu Gln Cys Ala Cys Ser Leu Lys Ala Met Ile Met Cys Gln Gly
   1505 1510 1515
   Cys Gly Ala Phe Cys His Asp Asp Cys Ile Gly Pro Ser Lys Leu
   1520 1525 1530
   Cys Val Leu Cys Leu Val Val Arg
   1535 1540
<210> 3
   <211> 36
   <212> PRT
   <213> Homo sapiens
<400> 3
   Cys Ala Cys Ser Leu Lys Ala Met Ile Met Cys Gln Gly Cys Gly Ala
   1 5 10 15
   Phe Cys His Asp Asp Cys Ile Gly Pro Ser Lys Leu Cys Val Leu Cys
   20 25 30
   Leu Val Val Arg
   35
<210> 4
   <211> 22
   <212> DNA
   <213> Artificial Sequence
<220>
   <223> Primer ASXL1 ex1 F
<400> 4
   tgacctctga ccccgtggtt at 22
<210> 5
   <211> 25
   <212> DNA
   <213> Artificial sequence
<220>
   <223> Primer ASXL1 Ex1 R
<400> 5
   ggctgtcccg ttcttaaagg aagat 25
<210> 6
   <211> 24
   <212> DNA
   <213> Artificial Sequence
<220>
   <223> Primer ASXL1 Ex2 F
<400> 6
   tcaccagcgg tacctcatag cata 24
<210> 7
   <211> 24
   <212> DNA
   <213> Artificial sequence
<220>
   <223> Primer ASXL1 Ex2 R
<400> 7
   tctcactacc agacacagaa gcac 24
<210> 8
   <211> 25
   <212> DNA
   <213> Artificial Sequence
<220>
   <223> Primer ASXL1 Ex3 F
<400> 8
   gaatgagttg gagggattag gtctt 25
<210> 9
   <211> 24
   <212> DNA
   <213> Artificial Sequence
<220>
   <223> Primer ASXL1 Ex3 R
<400> 9
   ttcatggccc ctattccaga ccta 24
<210> 10
   <211> 20
   <212> DNA
   <213> Artificial sequence
<220>
   <223> Primer ASXL1 Ex4-5 F
<400> 10
   cacctgagtt gtaccttgct 20
<210> 11
   <211> 24
   <212> DNA
   <213> Artificial Sequence
<220>
   <223> Primer ASXL1 Ex4-5 R
<400> 11
   gggatcttgc cttatacctg tcat 24
<210> 12
   <211> 24
   <212> DNA
   <213> Artificial sequence
<220>
   <223> Primer ASXL1 Ex6 F
<400> 12
   ctgcagttga cttgggctct cttt 24
<210> 13
   <211> 21
   <212> DNA
   <213> Artificial sequence
<220>
   <223> Primer ASXL1 Ex6 R
<400> 13
   tcatccattg gcagggctct t 21
<210> 14
   <211> 24
   <212> DNA
   <213> Artificial Sequence
<220>
   <223> Primer ASXL1 Ex7 F
<400> 14
   gctgggagaa atgagcttgt ctga 24
<210> 15
   <211> 22
   <212> DNA
   <213> Artificial Sequence
<220>
   <223> Primer ASXL1 Ex7 R
<400> 15
   aatagagggc cacccaagaa gt 22
<210> 16
   <211> 24
   <212> DNA
   <213> Artificial sequence
<220>
   <223> Primer ASXL1 Ex8-9 F
<400> 16
   ggtgctgtca ccaaagtttt ccca 24
<210> 17
   <211> 24
   <212> DNA
   <213> Artificial sequence
<220>
   <223> Primer ASXL1 Ex8-9 R
<400> 17
   aaccctttga gaagagcgag cttg 24
<210> 18
   <211> 22
   <212> DNA
   <213> Artificial sequence
<220>
   <223> Primer ASXL1 Ex10 F
<400> 18
   actgggagat tcagctgtcc at 22
<210> 19
   <211> 23
   <212> DNA
   <213> Artificial sequence
<220>
   <223> Primer ASXL1 Ex10 R
<400> 19
   tatcccctca aaaccccaga cca 23
<210> 20
   <211> 24
   <212> DNA
   <213> Artificial sequence
<220>
   <223> Primer ASXL1 Ex11 F
<400> 20
   ggcctgaaac tgatggctgt gatt 24
<210> 21
   <211> 24
   <212> DNA
   <213> Artificial Sequence
<220>
   <223> Primer ASXL1 Ex11 R
<400> 21
   tgaccctcaa agaaaacctg gctc 24
<210> 22
   <211> 22
   <212> DNA
   <213> Artificial Sequence
<220>
   <223> Primer ASXL1 EX12-1 F
<400> 22
   aggtcagatc acccagtcag tt 22
<210> 23
   <211> 24
   <212> DNA
   <213> Artificial sequence
<220>
   <223> Primer ASXL1 EX12-1 R
<400> 23
   tagcccatct gtgagtccaa ctgt 24
<210> 24
   <211> 24
   <212> DNA
   <213> Artificial Sequence
<220>
   <223> Primer ASXL1 EX12-2 F
<400> 24
   agaggacctg ccttctctga gaaa 24
<210> 25
   <211> 24
   <212> DNA
   <213> Artificial sequence
<220>
   <223> Primer ASXL1 Ex12-2 R
<400> 25
   ttcgatggga tgggtatcca atgc 24
<210> 26
   <211> 22
   <212> DNA
   <213> Artificial Sequence
<220>
   <223> Primer ASXL1 EX12-3 F
<400> 26
   acttgaaaac caaggctctc gt 22
<210> 27
   <211> 24
   <212> DNA
   <213> Artificial sequence
<220>
   <223> Primer ASXL1 EX12-3 R
<400> 27
   gcaaccatcc catctgtcct tgta 24
<210> 28
   <211> 24
   <212> DNA
   <213> Artificial Sequence
<220>
   <223> Primer ASXL1 EX12-4 F
<400> 28
   ggtggacaag gatgagaaac ccaa 24
<210> 29
   <211> 24
   <212> DNA
   <213> Artificial Sequence
<220>
   <223> Primer ASXL1 EX12-4 R
<400> 29
   tgtcctgtga catagcacgg actt 24
<210> 30
   <211> 25
   <212> DNA
   <213> Artificial Sequence
<220>
   <223> Primer ASXL1 EX12-5 F
<400> 30
   tggattccaa agagcagttc tcttc 25
<210> 31
   <211> 24
   <212> DNA
   <213> Artificial Sequence
<220>
   <223> Primer ASXL1 EX12-5 R
<400> 31
   catgacaaag ggcatccctt ccaa 24
<210> 32
   <211> 24
   <212> DNA
   <213> Artificial Sequence
<220>
   <223> Primer ASXL1 Ex12-6 F
<400> 32
   acaggaaagc tactgggcat agtc 24
<210> 33
   <211> 24
   <212> DNA
   <213> Artificial sequence
<220>
   <223> Primer ASXL1 EX12-6 R
<400> 33
   caagagtgct cctgcctaaa gagt 24